# EUROPEAN PATENT SPECIFICATION

(11) **EP 2 150 546 B1**
(45) Date of publication and mention of the grant of the patent: **19.01.2011**
(21) Application number: 08736964.1
(22) Date of filing: 14.04.2008
(51) Int. Cl.: C07D 403/04, C07D 401/14, C07D 417/14, A61K 31/506, A61P 35/00

(54) **PYRIMIDINE DERIVATIVES AS INHIBITORS OF PHOSPHATIDYLINOSITOL-3-KINASE**
PYRIMIDINDERIVATE ALS INHIBITOREN VON PHOSPHATIDYLINOSITOL-3-KINASE
DÉRIVÉS DE PYRIMIDINE COMME INHIBITEURS DE LA PHOSPHATIDYLINOSITOL-3-KINASE

(30) Priority: 12.04.2007 GB 0707088; 19.04.2007 GB 0707611
(43) Date of publication of application: 10.02.2010
(73) Proprietor: F. Hoffmann-La Roche AG, 4070 Basel (CH); The Institute of Cancer Research: Royal Cancer Hospital, London SW7 3RP (GB)
(72) Inventor: GOLDSMITH, Paul, John, Slough Berkshire SL1 4NL (GB); HANCOX, Timothy, Colin, Slough Berkshire SL1 4NL (GB); PEGG, Neil, Anthony, Slough Berkshire SL1 4NL (GB); SHUTTLEWORTH, Stephen, Joseph, Slough Berkshire SL1 4NL (GB); DECHAUX, Elsa, Amandine, Harlow Essex CM19 5TR (GB); PRICE, Stephen, Harlow Essex CM19 5TR (GB); LARGE, Jonathan, Martin, Sutton Surrey SM2 5NG (GB); MCDONALD, Edward, Sutton Surrey SM2 5NG (GB)
(74) Representative: Keen, Celia Mary
(86) International application number: PCT/GB2008/001301
(87) International publication number: WO 2008/125839

(56) References cited:
- EP-A- 1 277 738
- WO-A-2004/048365
- WO-A-2005/000404
- WO-A-2006/060194
- WO-A-2007/042810

## Description

### Field of the Invention

The present invention relates to pyrimidine compounds and to their use as inhibitors of phosphatidylinositol 3-kinase (P13K).

### Background to the Invention

Phosphatidylinositol (hereinafter abbreviated as "PI") is one of a number of phospholipids found in cell membranes. In recent years it has become clear that PI plays an important role in intracellular signal transduction. In the late 1980s, a PI3 kinase (PI3K) was found to be an enzyme which phosphorylates the 3-position of the inositol ring of phosphatidylinositol (D. Whitman et al, 1988, Nature, 332, 664).

PI3K was originally considered to be a single enzyme, but it has now been clarified that a plurality of subtypes are present in PI3K. Each subtype has its own mechanism for regulating activity. Three major classes of PI3Ks have been identified on the basis of their *in vitro* substrate specificity (B. Vanhaesebroeck,1997, Trend in Biol. Sci, 22, 267). Substrates for class I PI3Ks are PI, PI 4-phosphate (PI4P) and PI 4,5-biphosphate (PI (4,5)P2). Class I PI3Ks are further divided into two groups, class Ia and class Ib, in terms of their activation mechanism. Class Ia PI3Ks include PI3K p110α, p110β and p110δ subtypes, which transmit signals from tyrosine kinase-coupled receptors. Class Ib PI3K includes a p110γ subtype activated by a G protein-coupled receptor. PI and PI(4)P are known as substrates for class II PI3Ks. Class II PI3Ks include PI3K C2α, C2β and C2γ subtypes, which are characterized by containing C2 domains at the C terminus. The substrate for class III PI3Ks is PI only.

In the PI3K subtypes, the class Ia subtype has been most extensively investigated to date. The three subtypes of class Ia are heterodimers of a catalytic 110 kDa subunit and regulatory subunits of 85 kDa or 55 kDa. The regulatory subunits contain SH2 domains and bind to tyrosine residues phosphorylated by growth factor receptors with a tyrosine kinase activity or oncogene products, thereby inducing the PI3K activity of the p110 catalytic subunit which phosphorylates its lipid substrate. Thus, the class Ia subtypes are considered to be associated with cell proliferation and carcinogenesis, immune disorders and conditions involving inflammation.

WO 01/083456 describes a series of condensed heteroaryl derivatives which have activity as inhibitors of PI3 K and which suppress cancer cell growth.

WO 2004/048365 describes trisubstituted pyrimidines which are inhibitors of PI3 kinase and the use of these compounds in treating cancer.

W0 2005/000404 relates to a series of pyrimidine compounds that are used to treat or prevent disorders associated with excessive bone loss, including certain inflammatory and cancer-related conditions.

WO 2006/060194 relates to pyrimidine compounds which are useful for treating inflammatory and immune disorders.

WO 2007/042810 describes pyrimidine derivatives which are inhibitors of PI3 kinase and which may therefore be used to treat a variety of disorders including cancer, immune disorders and inflammation.

### Summary of the Invention

It has now been found that a series of novel pyrimidine compounds have activity as inhibitors of PI3K. The compounds exhibit selectivity for class Ia PI3Ks over class Ib, in particular for the p110δ subtype. Accordingly, the present invention provides a compound which is a pyrimidine of formula (I): 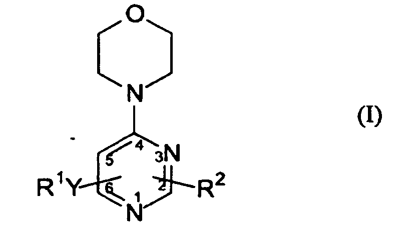 wherein
R² is bonded at ring position 2 and -YR¹ is bonded at ring position 5 or 6, or YR¹ is bonded at ring position 2 and R² is bonded at ring position 6;
R² is an indol-4-yl group which is substituted at the 5- or 6-position; either:
(a) Y is selected from -O-(CH₂)ₙ-, -NH-(CH₂)ₙ-, -NHC(O)-(CH₂)ₙ- and -C(O)NH-(CH₂)ₙ- wherein n is 0 or an integer of I to 3, and R¹ is selected from an unsaturated 5- to 12-membered carbocyclic or heterocyclic group which is unsubstituted or substituted and a group -NR³R⁴ wherein R³ and R⁴, which are the same or different, are each independently selected from H, C₁-C₆ alkyl which is unsubstituted or substituted, C₃ - C₁₀ cycloalkyl which is unsubstituted or substituted, -C(O)R, -C(O)N(R)₂ and -S(O)ₘR, or R³ and R⁴ together form, with the nitrogen atom to which they are attached, a saturated 5-, 6- or 7-membered N-containing heterocyclic group which is unsubstituted or substituted;
(b) Y is a direct bond and R¹ is selected from an unsaturated 5- to 12-membered carbocyclic or heterocyclic group which is unsubstituted or substituted, and a group -NR³R⁴ wherein R³ and R⁴, which are the same or different, are each independently selected from H, C₁-C₆ alkyl which is unsubstituted or substituted, C₃-C₁₀ cycloalkyl which is unsubstituted or substituted, -C(O)R, -C(O)N(R)₂ and -S(O)ₘR;
R is selected from H, C₁-C₆ alkyl, C₃-C₁₀ cycloalkyl and a 5- to 12-membered aryl or heteroaryl group, which group is unsubstituted or substituted; and
m is 1 or 2;
or a pharmaceutically acceptable salt thereof.

### Detailed description of the Invention

A C₁-C₆ alkyl group is linear or branched. A C₁-C₆ alkyl group is typically a C₁-C₄ alkyl group, for example a methyl, ethyl, propyl, n-butyl, sec-butyl or tert-butyl group. A C₁-C₆ alkyl group is unsubstituted or substituted, typically by one or more groups Z or R⁷ as defined below. Typically it is C₁-C₄ alkyl, for example methyl, ethyl, i-propyl, n-propyl, t-butyl, s-butyl or n-butyl.

Z is selected from H, halo, unsubstituted C₁-C₆ alkyl, -OR, -SR, CH₂OR, -CF₃, - (halo)-C₁-C₆ alkyl, -(C(R⁸)₂)_{q}O-(halo)-C₁-C₆ alkyl, -CO₂R, -(C(R⁸)₂)_{q}CO₂R, -(C(R⁸)₂)_{q}COR, CF₂OH, CH(CF₃)OH, C(CF₃)₂OH, -(CH₂)_{q}OR, -(C(R⁸)₂)_{q}OR, -(CH₂)_{q}NR₂, -(C(R⁸)₂)_{q}NR₂, - C(O)N(R)₂, -(C(R⁸)₂)_{q}CONR₂ , -NR₂, -(C(R⁸)₂)_{q}NR₂, -NRC(O)R, -(C(R⁸)₂)_{q}NRC(O)OR, - S(O)ₘR, -S(O)ₘN(R)₂, -(C(R⁸)₂)_{q}S(O)ₘN(R)₂, -OC(O)R, -(C(R⁸)₂)_{q}OC(O)R, -OC(O)N(R)₂, - (C(R⁸)₂)_{q}OC(O)N(R)₂, -(C(R⁸)₂)_{q}OC(O)NR₂, -NRS(O)ₘR, -(C(R⁸)₂)_{q}NRS(O)ₘR, - NRC(O)N(R)₂, -(C(R⁸)₂)_{q}NRC(O)N(R)₂, CN, -NO₂ and a 5- to 12-membered aryl or heteroaryl group, which group is unsubstituted or substituted, wherein each R is independently selected from H, C₁-C₆ alkyl, C₃-C₁₀ cycloalkyl and a 5- to 12-membered aryl or heteroaryl group, the group being unsubstituted or substituted, m is 1 or 2 and q is 0, 1 or 2.

R⁷ is selected from C₁-C₆ alkoxy, OR⁸, SR⁸, S(O)ₘR⁸, nitro, CN, halogen, -C(O)R⁸, - CO₂R⁸, -C(O)N(R⁸)₂ and -N(R⁸)₂,

R⁸, each of which is the same or different when more than one is present in a given substituent, is selected from H, C₁-C₆ alkyl and C₃-C₁₀cycloalkyl, and m is 1 or 2.

A halogen or halo group is F, Cl, Br or I. Preferably it is F, Cl or Br. A C₁-C₆ alkyl group substituted by halogen may be denoted by the term "halo-C₁-C₆ alkyl", which means an alkyl group in which one or more hydrogens is replaced by halo. A halo-C₁-C₆ alkyl group preferably contains one, two or three halo groups. A preferred example of such a group is trifluoromethyl.

A C₁-C₆ alkoxy group is linear or branched. It is typically a C₁-C₄ alkoxy group, for example a methoxy, ethoxy, propoxy, i-propoxy, n-propoxy, n-butoxy, sec-butoxy or tert-butoxy group. A C₁-C₆ alkoxy group is unsubstituted or substituted, typically by one or more groups Z or R⁷ as defined above.

A C₃-C₁₀ cycloalkyl group may be, for instance, C₃-C₈ cycloalkyl such as cyclopropyl, cyclobutyl, cyclopentyl, cyclohexyl, or cycloheptyl. Typically it is C₃-C₆ cycloalkyl. A C₃-C₁₀ cycloalkyl group is unsubstituted or substituted, typically by one or more groups Z or R⁷ as defined above.

A C₁-C₆ acyl group is a group -C(O)Alk in which Alk is C₁-C₆ alkyl as defined above. It is, for instance, formyl, acetyl or propionyl.

A saturated 5-, 6-, or 7-membered N-containing heterocyclic group typically contains one nitrogen atom and either an additional N atom or an O atom, or no additional heteroatoms. It may be, for example, piperidine, piperazine, morpholine, pyrrolidine or homopiperazine.

The saturated 5-, 6-, or 7-membered N-containing heterocyclic group is unsubstituted or substituted on one or more ring carbon atoms and/or on any additional N atom present in the ring. Examples of suitable substituents include one or more groups Z or R⁷ as defined above, and a C₁-C₆ alkyl group which is unsubstituted or substituted by a group Z or R⁷ as defined above. When the ring is piperazine it is typically unsubstituted or substituted, typically on the second ring nitrogen atom, by -C(O)R⁸, -C(O)N(R⁸)₂ or -S(O)ₘR⁸, or by C₁-C₆ alkyl which is unsubstituted or substituted by C₁-C₆ alkoxy or OH.

An unsaturated 5- to 12-membered carbocyclic group is a 5-, 6-, 7-, 8-, 9-, 10, 11- or 12-membered carbocyclic ring containing at least one unsaturated bond. It is a monocyclic or fused bicyclic ring system. The group is aromatic or non-aromatic, for instance a 5- to 12-membered aryl group. Examples include phenyl, naphthyl, indanyl, indenyl and tetrahydronaphthyl groups. The group is unsubstituted or substituted, typically by one or more groups Z or R⁷ as defined above.

An aryl group is a 5- to 12-membered aromatic carbocyclic group. It is monocyclic or bicyclic. Examples include phenyl and naphthyl groups. The group is unsubstituted or substituted, for instance by a group Z or R⁷ as defined above.

An unsaturated 5- to 12-membered heterocyclic group is a 5-, 6-, 7-, 8-, 9-, 10, 11- or 12-membered heterocyclic ring containing at least one unsaturated bond and at least one heteroatom selected from O, N and S. It is a monocyclic or fused bicyclic ring system. The group is aromatic or non-aromatic, for instance heteroaryl. The group may be, for example, furan, thiophene, pyrrole, pyrrolopyrazine, pyrrolopyrimidine, pyrrolopyridine, pyrrolopyridazine, indole, isoindole, pyrazole, pyrazolopyrazine, pyrazolopyrimidine, pyrazolopyridine, pyrazolopyridazine, imidazole, imidazopyrazine, imidazopyrimidine, imidazopyridine, imidazopyridazine, benzimidazole, benzodioxole, benzodioxine, benzoxazole, benzothiophene, benzothiazole, benzofuran, indole, indolizinyl, isoxazole, oxazole, oxadiazole, thiazole, isothiazole, thiadiazole, dihydroimidazole, dihydrobenzofuran, dihydrodioxinopyridine, dihydropyrrolopyridine, dihydrofuranopyridine, dioxolopyridine, pyridine, quinoline, isoquinoline, quinazoline, quinoxaline, tetrahydrobenzofuran, tetrahydroquinoline, tetrahydroisoquinoline, 5,6,7,8-tetrahydro-imidazo[1,5-a]pyrazine, 5,6,7,8-tetrahydro-imidazo[1,2-a]pyrazine, thienopyrazine, pyran, pyrimidine, pyridazine, pyrazine, triazine, triazole or tetrazole. The group is unsubstituted or substituted, typically by one or more groups Z or R⁷ as defined above.

Heteroaryl is a 5- to 12-membered aromatic heterocyclic group which contains 1, 2, 3, or 4 heteroatoms selected from O, N and S. It is monocyclic or bicyclic. Typically it contains one N atom and 0, 1, 2 or 3 addditional heteroatoms selected from O, S and N. It may be, for example, selected from the heteroaryl groups in the above list of options for a 5 to 12-menbered heterocyclic group.

In the definition of R¹ this heterocyclic group is typically selected from pyridine, thiophene and pyrrole. Most typically it is pyridine, for instance a pyrid-2-yl, pyrid-3-yl or pyrid-4-yl group.

When R¹ is an unsaturated 5- to 12-membered carbocyclic group it is typically an aromatic carbocyclic group such as phenyl or naphthyl. When R¹ is an unsaturated 5- to 12-membered heterocyclic group it is typically pyridyl, for instance a pyrid-2-yl, pyrid-3-yl or pyrid-4-yl group. When R¹ is a saturated 5-, 6- or 7-membered N-containing heterocyclic group it is typically a 6-membered such heterocyclic group, for instance piperidyl or morpholinyl. The group R¹ is unsubstituted or substituted, for instance by a group Z or R⁷ as defined above.

R² is an indol-4-yl group which is substituted at the 5- or 6-position. Examples of suitable substituents include CN, halo, -C(O)NR₂, halo(C₁-C₆)alkyl, -SO₂R, -SO₂NR₂, and a 5-membered heteroaryl group containing 1, 2, 3 or 4 heteroatoms selected from O, N and S, wherein R is H or C₁-C₆ alkyl. Typically the substituent is an electron-withdrawing group.

The 5-membered heteroaryl group may be, for example, furan, thiophene, pyrrole, imidazole, pyrazole, triazole, tetrazole, oxazole, isoxazole, oxadiazole, thiazole, isothiazole, or thiadiazole.

More typically the indol-4-yl group is substituted at the 5- or 6-position by CN, halo, - C(O)NH₂, -CF₃, -SO₂Me, -SO₂NMe₂ or a 5-membered heteroaryl group as defined above. Most typically the indol-4-yl group is substituted at the 6-position by halo, in particular by F.

In one embodiment the pyrimidine is of formula (Ia): 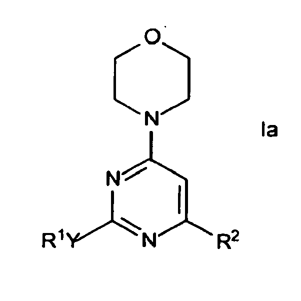 wherein R¹, R² and Y are as defined above for formula (I).

In formula (Ia) Y is typically selected from a direct bond, -C(O)NH(CH₂)₂-, -NHC(O)-, -NH-, -NH-CH₂-, -NH-(CH₂)₂-, -NH-(CH₂)₃-, -O-, -OCH₂- and -O(CH₂)₂-. R¹ is typically an aryl or heteroaryl group which is unsubstituted or substituted, for instance by a phenyl or pyridyl group. The phenyl group is unsubstituted or substituted, for instance by a group Z or a group R⁷ as defined above, for example by a halogen such as Cl or Br. The pyridyl group is unsubstituted or substituted by a group Z or R⁷ as defined above. The pyridyl group is typically a pyrid-3-yl or pyrid-4-yl group. R² is typically an indol-4-yl group substituted at the 5-position by halo or at the 6-position by halo, CN, CF₃, -CONH₂, - SO₂NMe₂ or -SO₂Me.

In a second embodiment the pyrimidine is of formula (Ib): 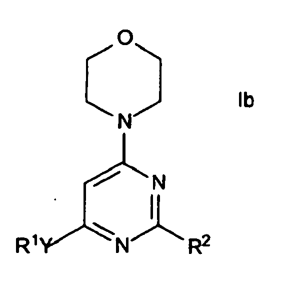 wherein R¹, R² and Y are as defined above for formula (I).

In formula (Ib), Y is typically a group -NH-(CH₂)ₙ- in which n is 1, 2 or 3. R¹ is typically an aryl or heteroaryl group which is unsubstituted or substituted, for instance a phenyl or pyridyl group. The phenyl group is unsubstituted or substituted, for instance by a group Z or a group R⁷ as defined above, for example by a halogen such as Cl or Br. The pyridyl group is unsubstituted or substituted by a group Z or R⁷ as defined above. The pyridyl group is typically a pyrid-3-yl or pyrid-4-yl group.

In a third embodiment the pyrimidine is of formula (Ic): 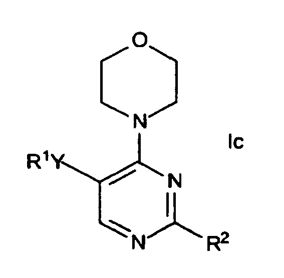 wherein R¹, R² and Y are as defined above for formula (I).

Specific examples of compounds of the invention include those listed in the following Table 1:

**Table 1**

| Compound No. | Structure | Name |
|---|---|---|
| 1 | 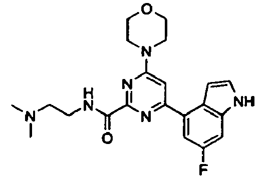 | 4-(6-Fluoro-1*H*-indol-4-yl)-6-morpholin-4-yl-pyrimidine-2-carboxylic acid (2-dimethylamino-ethyl)-amide |
| 2 | 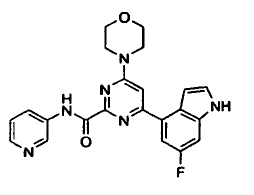 | 4-(6-Fluoro-1*H*-indol-4-yl)-6-morpholin-4-yl-pyrimidine-2-carboxylic acid pyridin-3-ylamide |
| 3 | 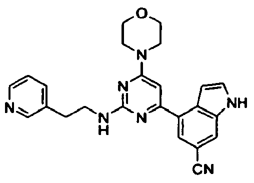 | 4-[6-Morpholin-4-yl-2-(2-pyridin-3-yl-ethylamino)-pyrimidin-4-yl]-1H-indole-6-carbonitrile |
| 4 | 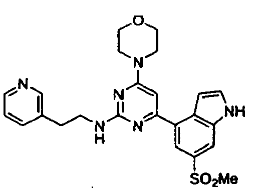 | [4-(6-Methanesulfonyl-1H-indol-4-yl)-6-morpholin-4-yl-pyrimidin-2-yl]-(2-pyridin-3-yl-ethyl)-amine |
| 5 | 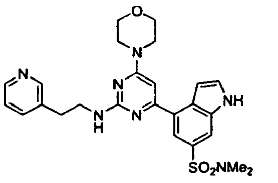 | 4-[6-Morpholin-4-yl-2-(2-pyridin-3-yl-ethylamino)-pyrimidin-4-yl]-1H-indole-6-sulfonic acid dimethylamide |
| 6 | 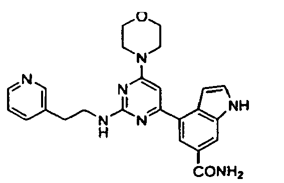 | 4-[6-Morpholin-4-yl-2-(2-pyridin-3-yl-ethylamino)-pyrimidin-4-yl]-1H-indole-6-carboxylic acid amide |
| 7 | 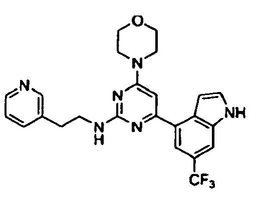 | [4-Morpholin-4-yl-6-(6-trifluoromethyl-1H-indol-4-yl)-pyrimidin-2-yl]-(2-pyridin-3-yl-ethyl)-amine |
| 8 | 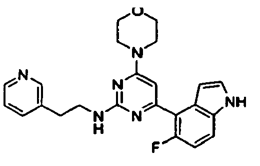 | [4-(5-Fluoro-1H-indol-4-yl)-6-morpholin-4-yl-pyrimidin-2-yl]-(2-pyridin-3-yl-ethyl)-amine |
| 9 | 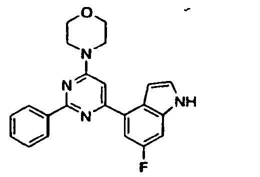 | 6-Fluoro-4-(6-morpholin-4-yl-2-phenyl-pyrimidin-4-yl)-1H-indole |
| 10 | 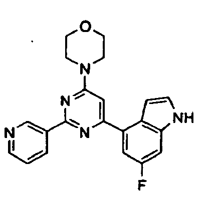 | 6-Fluoro-4-(6-morpholin-4-yl-2-pyridin-3-yl-pyrimidin-4-yl)-1H-indole |
| 11 | 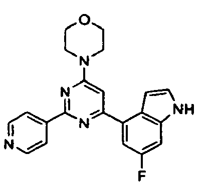 | 6-Fluoro-4-(6-morpholin-4-yl-2-pyridin-4-yl-pyrimidin-4-yl)-1H-indole |
| 12 | 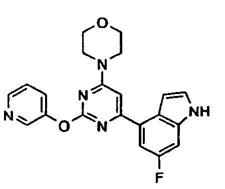 | 6-Fluoro-4-[6-morpholin-4-yl-2-(pyridin-3-yloxy)-pyrimidin-4-yl]-1H-indole |
| 13 | 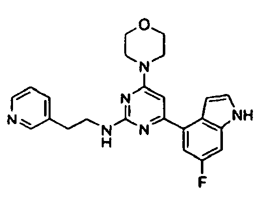 | [4-(6-Fluoro-1H-indol-4-yl)-6-morpholin-4-yl-pyrimidin-2-yl]-(2-pyridin-3-yl-ethyl)-amine |
| 14 | 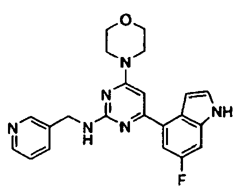 | [4-(6-Fluoro-1H-indol-4-yl)-6-morpholin-4-yl-pyrimidin-2-yl]-pyridin-3-ylmethyl-amine |
| 15 | 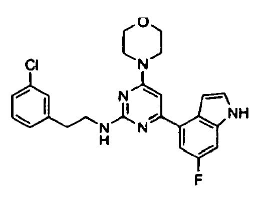 | [2-(3-Chloro-phenyl)-ethyl]-[4-(6-fluoro-1H-indol-4-yl)-6-morpholin-4-yi-pyrimidin-2-yl]-amine |
| 16 | 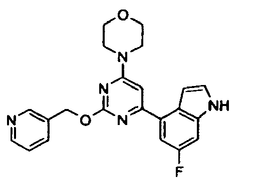 | 6-Fluoro-4-[6-morpholin-4-yl-2-(pyridin-3-ylmethoxy)-pyrimidin-4-yl]-1H-indole |
| 17 | 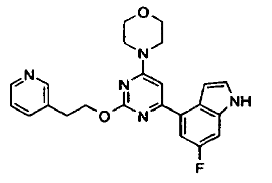 | 6-Fluoro-4-[6-morpholin-4-yl-2-(2-pyridin-3-yl-ethoxy)-pyrimidin-4-yl]-1H-indole |
| 18 | 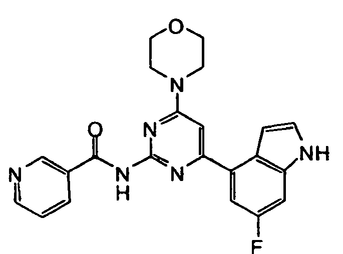 | *N*-[4-(6-Fluoro-1H-indol-4-yl)-6-morpholin-4-yl-pyrimidin-2-yl]-nicotinamide |
| 19 | 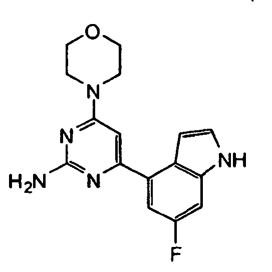 | 4-(6-Fluoro-1H-indol-4-yl)-6-morpholin-4-yl-pyrimidin-2-ylamine |
| 20 | 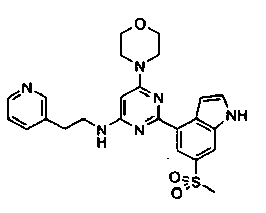 | [2-(6-Methanesulfonyl-1H-indol-4-yl)-6-morpholin-4-yl-pyrimidin-4-yl]-(2-pyridin-3-yl-ethyl)-amine |
| 21 | 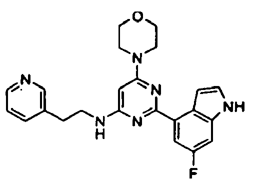 | [2-(6-Fluoro-1H-indol-4-yl)-6-morpholin-4-yl-pyrimidin-4-yl]-(2-pyridin-3-yl-ethyl)-amine |
| 22 | 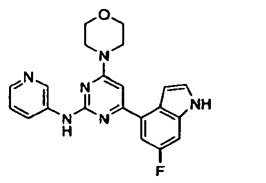 | [4-(6-Fluoro-1H-indol-4-yl)-6-morpholin-4-yl-pyrimidin-2-yl]-pyridin-3-yl-amine |
| 23 | 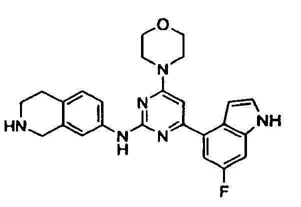 | [4-(6-Fluoro-1*H*-indol-4-yl)-6-morpholin-4-yl-pyrimidin-2-yl]-(1,2,3,4-tetrahydro-isoquinolin-7-yl)-amine |
| 24 | 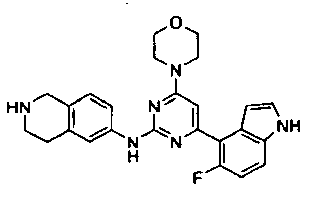 | [4-(5-Fluoro-1*H*-indol-4-yl)-6-morpholin-4-yl-pyrimidin-2-yl]-(1,2,3,4-tetrahydro-isoquinolin-6-yl)-amine |
| 25 | 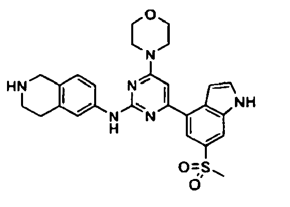 | [4-(6-Methanesulfonyl-1*H*-indol-4-yl)-6-morpholin-4-yl-pyrimidin-2-yl]-(1,2,3,4-tetrahydro-isoquinolin-6-yl)-amine |
| 26 | 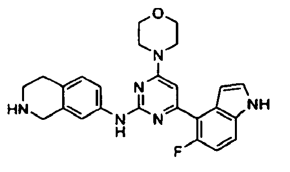 | [4-(5-Fluoro-1*H*-indol-4-yl)-6-morpholin-4-yl-pyrimidin-2-yl]-(1,2,3,4-tetrahydro-isoquinolin-7-yl)-amine |
| 27 | 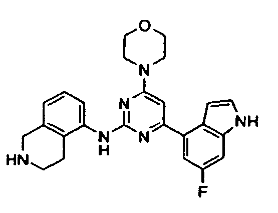 | [4-(6-Fluoro-1*H*-indol-4-yl)-6-morpholin-4-yl-pyrimidin-2-yl]-(1,2,3,4-tetrahydro-isoquinolin-5-yl)-amine |
| 28 | 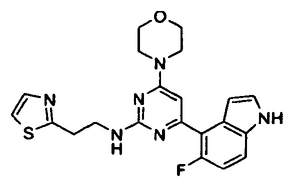 | [4-(5-Fluoro-1*H*-indol-4-yl)-6-morpholin-4-yl-pyrimidin-2-yl]-(2-thiazol-2-yl-ethyl)-amine |
| 29 | 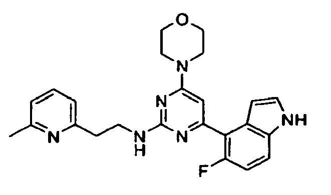 | [4-(5-Fluoro-1*H*-indol-4-yl)-6-morpholin-4-yl-pyrimidin-2-yl]-[2-(6-methyl-pyridin-2-yl)-ethyl]-amine |
| 30 | 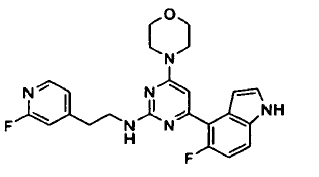 | [4-(5-Fluoro-1*H*-indol-4-yl)-6-morpholin-4-yl-pyrimidin-2-yl]-[2-(2-fluoro-pyridin-4-yl)-ethyl]-amine |
| 31 | 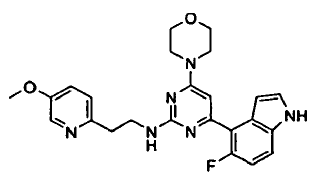 | [4-(5-Fluoro-1*H*-indol-4-yl)-6-morpholin-4-yl-pyrimidin-2-yl]-[2-(5-methoxy-pyridin-2-yl)-ethyl]-amine |
| 32 | 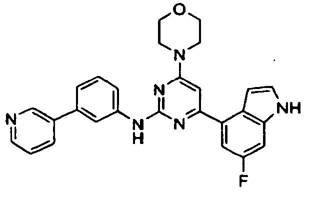 | 4-(6-Fluoro-1H-indol-4-yl)-6-morpholin-4-yl-pyrimidin-2-yl-(3-pyridin-3-yl-phenyl)-amine |

and the pharmaceutically acceptable salts thereof.

Pyrimidines of formula (I) may be converted into pharmaceutically acceptable salts, and salts may be converted into the free compound, by conventional methods. Pharmaceutically acceptable salts include salts of inorganic acids such as hydrochloric acid, hydrobromic acid and sulfuric acid, and salts of organic acids such as acetic acid, oxalic acid, malic acid, methanesulfonic acid, trifluoroacetic acid, benzoic acid, citric acid and tartaric acid. In the case of compounds of the invention bearing a free carboxy substituent, the salts include both the above-mentioned acid addition salts and the salts of sodium, potassium, calcium and ammonium. The latter are prepared by treating the free pyrimidine of formula (I), or the acid addition salt thereof, with the corresponding metal base or ammonia.

Pyrimidines of the invention may be produced by a process which comprises a palladium-mediated (Suzuki-type) cross-coupling reaction, typically as either the last step or as the penultimate step. When the Suzuki cross-coupling reaction is the last step, a pyrimidine of formula (I) may be produced by a process which comprises treating a compound of formula (IIa) or (IIb): 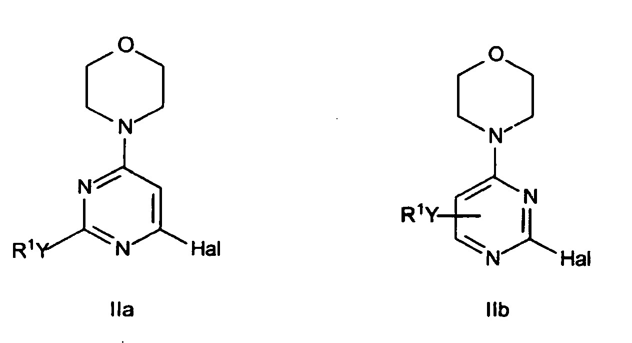 wherein R¹ and Y are as defined above and Hal is a halogen, with a boronic acid or ester thereof of formula R²B(OR¹⁵)₂, in which R² is as defined above and each R¹⁵ is H or C₁-C₆ alkyl or the two groups OR¹⁵ form, together with the boron atom to which they are attached, a pinacolato boronate ester group, in the presence of a Pd catalyst.

The intermediate compounds of formulae (IIa) and (IIb) are known compounds which can be obtained commercially or made by routine synthetic chemical techniques. For example, a compound of formula (IIa) in which Y is -NH-(CH₂)ₙ- or -NHC(O)-(CH₂)ₙ- , or of formula (IIb) wherein the moiety -YR¹ is at ring position 6 and Y is -NH-(CH₂)ₙ- or -NHC(O)-(CH₂)ₙ- , may be produced by a process which comprises treating a compound of formula (IIIa) or (IIIb): 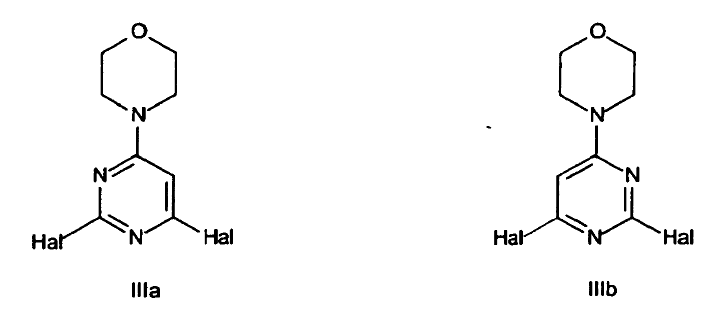 wherein each Hal is halogen, with an amine of formula HYR¹ in a solvent in the presence of a base or by a copper or palladium mediated coupling process.

A compound of formula (IIa) or (IIb) in which Y is a direct bond and R¹ is an unsaturated 5- to 12-membered carbocyclic or heterocyclic group which is unsubstituted or substituted may be produced by a process which comprises treating a compound of formula (IIIa) or (IIIb) as defined above with a boronic acid or ester thereof of formula R¹B(OR¹⁵)₂ in which R¹⁵ is as defined above in the presence of a Pd catalyst.

A compound of formula (IIa) or (IIb) in which Y is -C(O)NH-(CH₂)ₙ- may be produced by a process which comprises treating a compound of formula (IIIc) or (IIId): 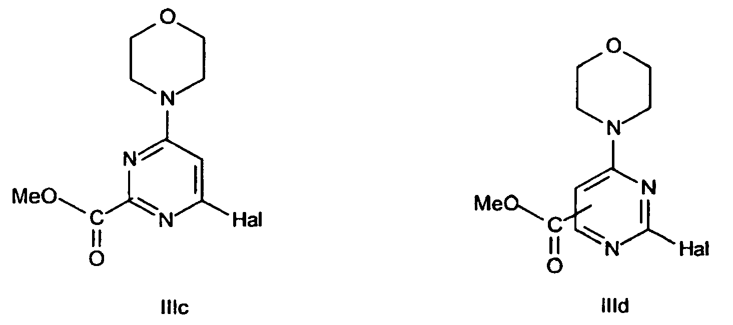 with an amine of formula R¹-(CH₂)ₙ-NH₂ in an inert solvent in the presence of Me₃Al.

When the palladium-mediated Suzuki cross-coupling reaction is the penultimate step, that step may comprise producing an intermediate compound of formula (IIc) or (IId): 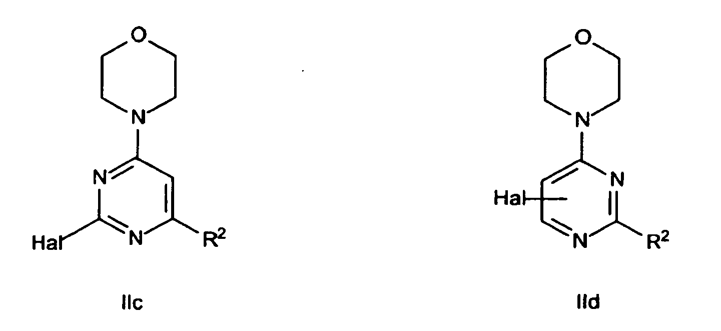 wherein R² is as defined above and Hal is a halogen, by treating a compound of formula (IIIa) or (IIIb) as defined above with a boronic acid or ester thereof of formula R²B(OR¹⁵)₂, in which R² is as defined above and each R¹⁵ is H or C₁-C₆ alkyl or the two groups OR¹⁵ form, together with the boron atom to which they are attached, a pinacolato boronate ester group, in the presence of a Pd catalyst.

The intermediate compounds of formulae (IIc) and (IId) may be converted to a pyrimidine of formula (I) as defined above in which Y is a direct bond and R¹ is a group - NR³R⁴ as defined above, by a process which comprises treating a compound of formula (IIc) or (IId) as defined above, typically a compound of formula (IId) in which Hal is bonded at ring position 6, with an amine of formula HNR³R⁴ in a solvent at an elevated temperature.

When the Suzuki cross-coupling is the penultimate step, that step may alternatively comprise producing an intermediate compound of formula (IIe) or (IIf): 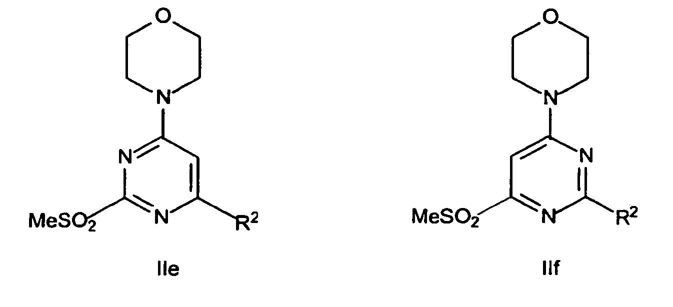 wherein R² is as defined above, by treating a compound of the following formula (IIIe) or (IIIf): 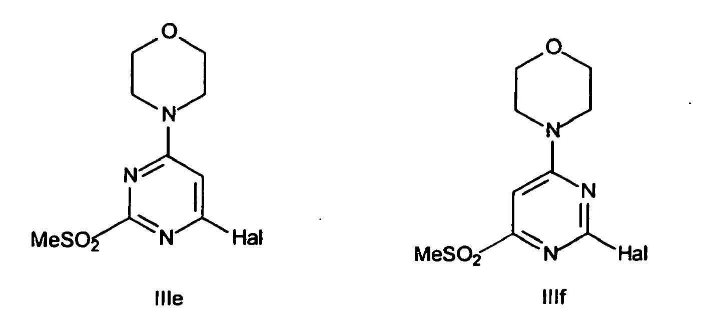 wherein Hal is a halogen with a boronic acid or ester thereof of formula R²B(OR¹⁵)₂, in which R² is as defined above and each R¹⁵ is H or C₁-C₆ alkyl or the two groups OR¹⁵ form, together with the boron atom to which they are attached, a pinacolato boronate ester group, in the presence of a Pd catalyst.

The intermediate compounds of formulae (IIe) and (IIf) may be converted to a pyrimidine of formula (I) as defined above in which Y is a direct bond and R¹ is a group - NR³R⁴ as defined above, by a process which comprises treating a compound of formula (IIe) or (IIf), typically a compound of formula (IIe), with an amine of formula HNR³R⁴ in a solvent at an elevated temperature.

A compound of formula (IIIe) or (IIIf) may be produced by a process which comprises oxidising a compound of the following formula (IVe) or (IVf):

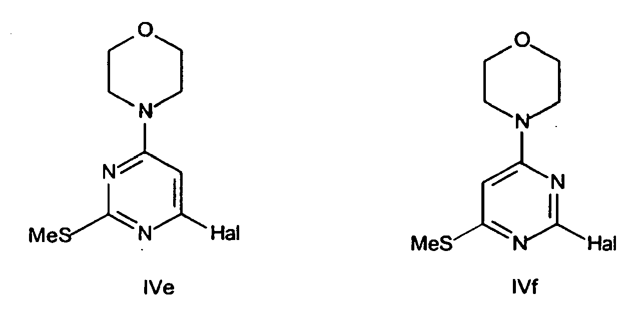

The oxidation may be performed by any suitable method for converting a group -S- to -S(O)₂-.

Compounds of the present invention have been found in biological tests to be inhibitors of PI3 kinase. The compounds are selective for class Ia PI3 kinases over class Ib. In general the compounds are selective for the p110δ isoform, for instance p110δ over p110γ.

A compound of the present invention may thus be used as an inhibitor of PI3 kinase, in particular of a class Ia PI3 kinase. Accordingly, the invention further provides a compound of the present invention as defined above for use in treating cancer, immune disorders, cardiovascular disease, viral infection, inflammation, metabolism/endocrine disorders or neurological disorders. Such diseases and disorders are discussed by Drees et al in Expert Opin. Ther. Patents (2004) 14(5):703 - 732. Examples of metabolism/endocrine disorders include diabetes and obesity. Examples of cancers which the present compounds can be used to treat include leukaemia, brain tumours, renal cancer, gastric cancer and cancer of the skin, bladder, breast, uterus, lung, colon, prostate, ovary and pancreas.

A compound of the present invention may be used as an inhibitor of PI3 kinase. A human or animal patient suffering from an immune disorder, cancer, cardiovascular disease, viral infection, inflammation, a metabolism/endocrine disorder or a neurological disorder, may thus be treated by a method comprising the administration thereto of a compound of the present invention as defined above. The condition of the patient may thereby be improved or ameliorated.

A compound of the present invention can be administered in a variety of dosage forms, for example orally such as in the form of tablets, capsules, sugar- or film-coated tablets, liquid solutions or suspensions or parenterally, for example intramuscularly, intravenously or subcutaneously. The compound may therefore be given by injection or infusion.

The dosage depends on a variety of factors including the age, weight and condition of the patient and the route of administration. Daily dosages can vary within wide limits and will be adjusted to the individual requirements in each particular case. Typically, however, the dosage adopted for each route of administration when a compound is administered alone to adult humans is 0.0001 to 50 mg/kg, most commonly in the range of 0.001 to 10 mg/kg, body weight, for instance 0.01 to 1 mg/kg. Such a dosage may be given, for example, from 1 to 5 times daily. For intravenous injection a suitable daily dose is from 0.0001 to 1 mg/kg body weight, preferably from 0.0001 to 0.1 mg/kg body weight. A daily dosage can be administered as a single dosage or according to a divided dose schedule.

A compound of the invention is formulated for use as a pharmaceutical or veterinary composition also comprising a pharmaceutically or veterinarily acceptable carrier or diluent. The compositions are typically prepared following conventional methods and are administered in a pharmaceutically or veterinarily suitable form. The compound may be administered in any conventional form, for instance as follows:
A) Orally, for example, as tablets, coated tablets, dragees, troches, lozenges, aqueous or oily suspensions, liquid solutions, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of sweetening agents, flavouring agents, colouring agents and preserving agents in order to provide pharmaceutically elegant and palatable preparations.
   Tablets contain the active ingredient in admixture with non-toxic pharmaceutically acceptable excipients which are suitable for the manufacture of tablets. These excipients may be for example, inert diluents, such as calcium carbonate, sodium carbonate, lactose, dextrose, saccharose, cellulose, corn starch, potato starch, calcium phosphate or sodium phosphate; granulating and disintegrating agents, for example, maize starch, alginic acid, alginates or sodium starch glycolate; binding agents, for example starch, gelatin or acacia; lubricating agents, for example silica, magnesium or calcium stearate, stearic acid or talc; effervescing mixtures; dyestuffs, sweeteners, wetting agents such as lecithin, polysorbates or lauryl sulphate. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. Such preparations may be manufactured in a known manner, for example by means of mixing, granulating, tableting, sugar coating or film coating processes.
   Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is present as such, or mixed with water or an oil medium, for example, peanut oil, liquid paraffin, or olive oil.
   Aqueous suspensions contain the active materials in admixture with excipients suitable for the manufacture of aqueous suspensions. Such excipients are suspending agents, for example, sodium carboxymethylcellulose, methylcellulose, hydroxypropylmethylcellulose, sodium alginate, polyvinylpyrrolidone gum tragacanth and gum acacia; dispersing or wetting agents may be naturally-occurring phosphatides, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides for example polyoxyethylene sorbitan monooleate.
   The said aqueous suspensions may also contain one or more preservatives, for example, ethyl or n-propyl p-hydroxybenzoate, one or more colouring agents, such as sucrose or saccharin.
   Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol.
   Sweetening agents, such as those set forth above, and flavouring agents may be added to provide a palatable oral preparation. These compositions may be preserved by this addition of an antioxidant such as ascorbic acid. Dispersible powders and granules suitable for preparation of an aqueous suspension by the addition of water provide the active ingredient in admixture with a dispersing or wetting agent, a suspending agent and one or more preservatives. Suitable dispersing or wetting agents and suspending agents are exemplified by those already mentioned above. Additional excipients, for example sweetening, flavouring and colouring agents, may also be present.
   The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally occuring phosphatides, for example soy bean lecithin, and esters or partial esters derived from fatty acids an hexitol anhydrides, for example sorbitan mono-oleate, and condensation products of the said partial esters with ethylene oxide, for example polyoxyethylene sorbitan monooleate. The emulsion may also contain sweetening and flavouring agents. Syrups and elixirs may be formulated with sweetening agents, for example glycerol, sorbitol or sucrose. In particular a syrup for diabetic patients can contain as carriers only products, for example sorbitol, which do not metabolise to glucose or which only metabolise a very small amount to glucose.
   Such formulations may also contain a demulcent, a preservative and flavouring and coloring agents.
B) Parenterally, either subcutaneously, or intravenously, or intramuscularly, or intrasternally, or by infusion techniques, in the form of sterile injectable aqueous or oleaginous suspensions. This suspension may be formulated according to the known art using those suitable dispersing of wetting agents and suspending agents which have been mentioned above. The sterile injectable preparation may also be a sterile injectable solution or suspension in a non-toxic paternally-acceptable diluent or solvent, for example as a solution in 1,3-butane diol.
   Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition fatty acids such as oleic acid find use in the preparation of injectables.
C) By inhalation, in the form of aerosols or solutions for nebulizers.
D) Rectally, in the form of suppositories prepared by mixing the drug with a suitable non-irritating excipient which is solid at ordinary temperature but liquid at the rectal temperature and will therefore melt in the rectum to release the drug. Such materials are cocoa butter and poly-ethylene glycols.
E) Topically, in the form of creams, ointments, jellies, collyriums, solutions or suspensions.

The invention will be further described in the Examples which follow:

### EXAMPLES

### General Synthetic Procedures

The following general schemes 1 to 8 are referred to in the Reference Examples as Examples which follow: 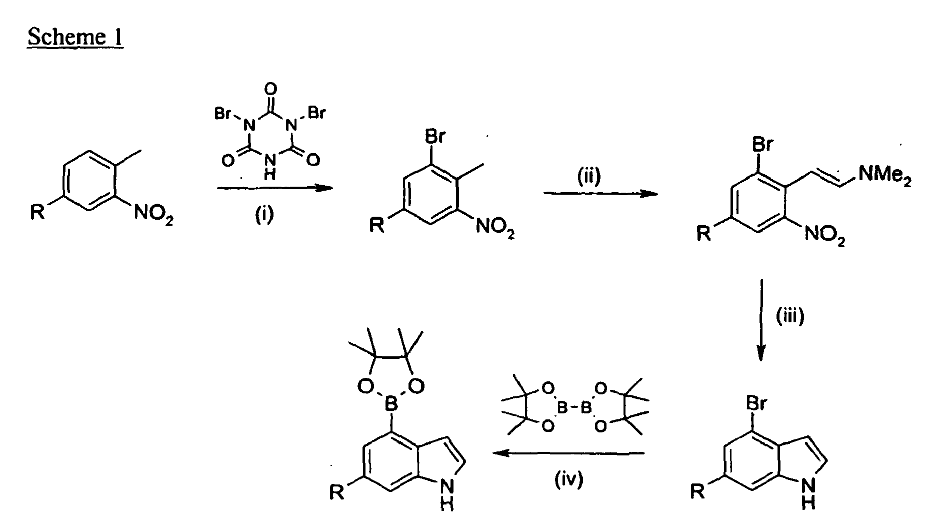 Conditions: (i) H₂SO₄, 21 h. (ii) Dioxane, DMF-DMA, 80°C, 24 h, 90°, 16 h. (iii) MeOH-THF Raney® Nickel, NH₂NH₂.H₂O, RT, 40 min. (iv) DMSO, KOAc, Pd(dppf)₂Cl₂ 80°C. 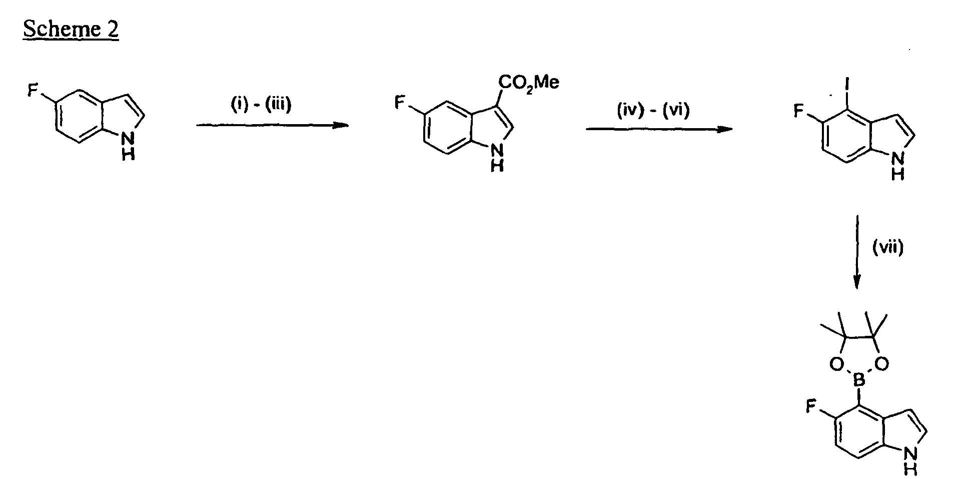 Conditions: (i) DMF, TFAA, 0°C. (ii) 10% aq NaOH, 100°C, 1h. (iii) MeOH, H₂SO₄, 65°C, 18 h. (iv) Tl(OCOCF₃)₃, TFA, RT, 2 h. (v) H₂O, KI, RT. (vi) MeOH, 40% aq NaOH, 65°C, 2 h. (vii) pinacol borane, Et₃N, Dioxane, Pd(OAc)₂, bis(cyclohexyl)phosphino-2-biphenyl, 80 °C, 30 min. 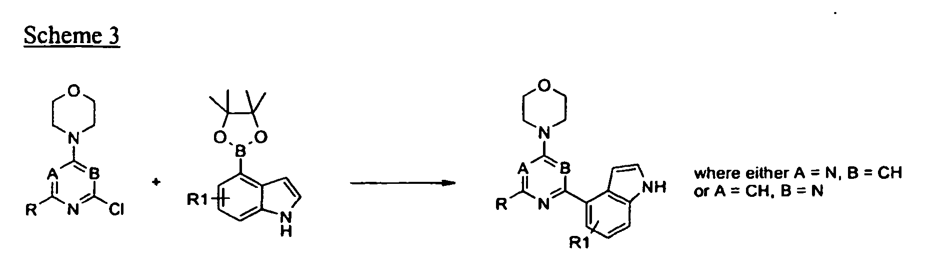 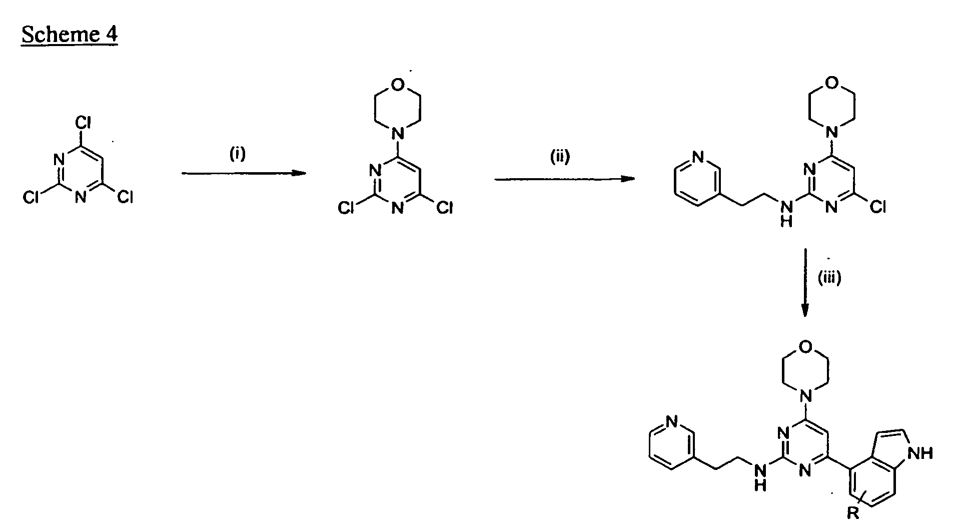 Conditions: (i) morpholine, DIPEA, dioxane, 0°C→RT, 24 h. (ii) 3-(2-aminoethyl)-pyridine, DIPEA, dioxane, RT→70°C, 40 h. (iii) boronate ester, PdCl₂(PCy₃)₂, K₃PO₄, dioxane, microwave 125°C, 30 - 90 minutes. 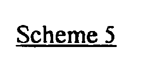 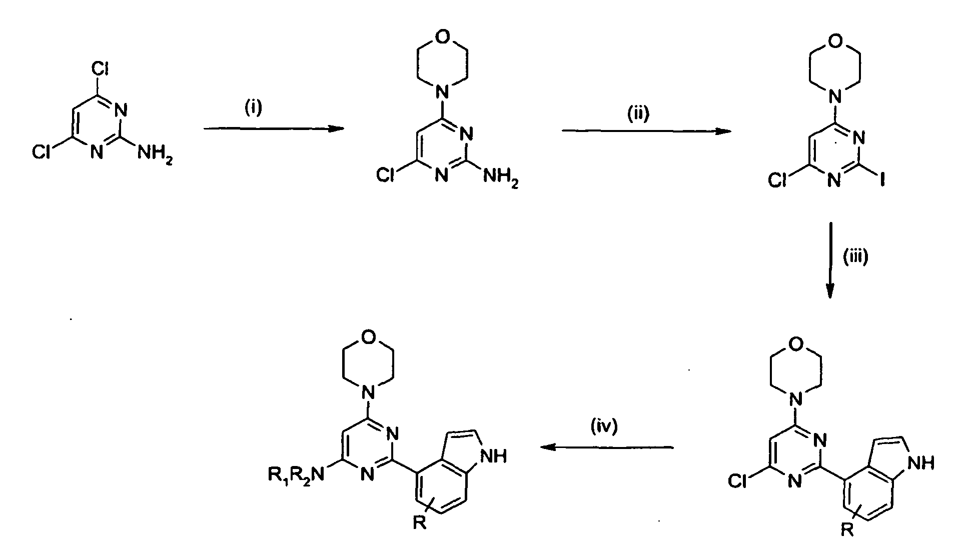 Conditions: (i) morpholine, Et₃N, MeOH, 65°C, 18 h. (ii) isoamylnitrite, CH₂I₂, CuI, THF, 67°C, 1 h. (iii) Suzuki coupling, (iv) R1R2NH, NMP, 150°C, 18 h. 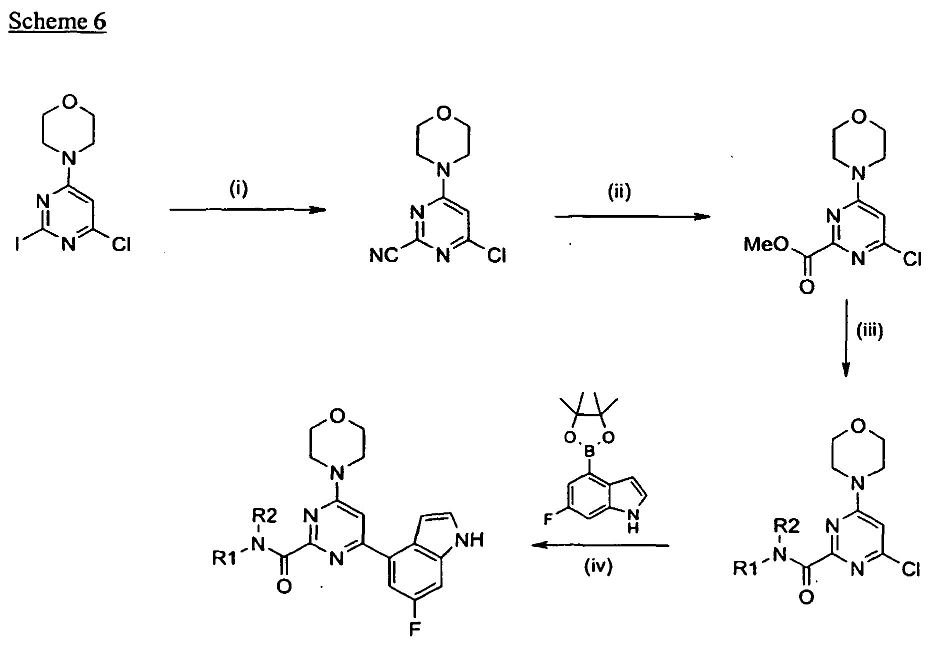 Conditions: (i) Pd₂(dba)₃, dppf, Zn, Zn(CN)₂, DMA, 120°C, 45 min. (ii) HCl, MeOH, 65°C, 3 h. (iii) R1R2NH, toluene, Me₃Al, 0°C→R, 16 h. (iv) Suzuki coupling 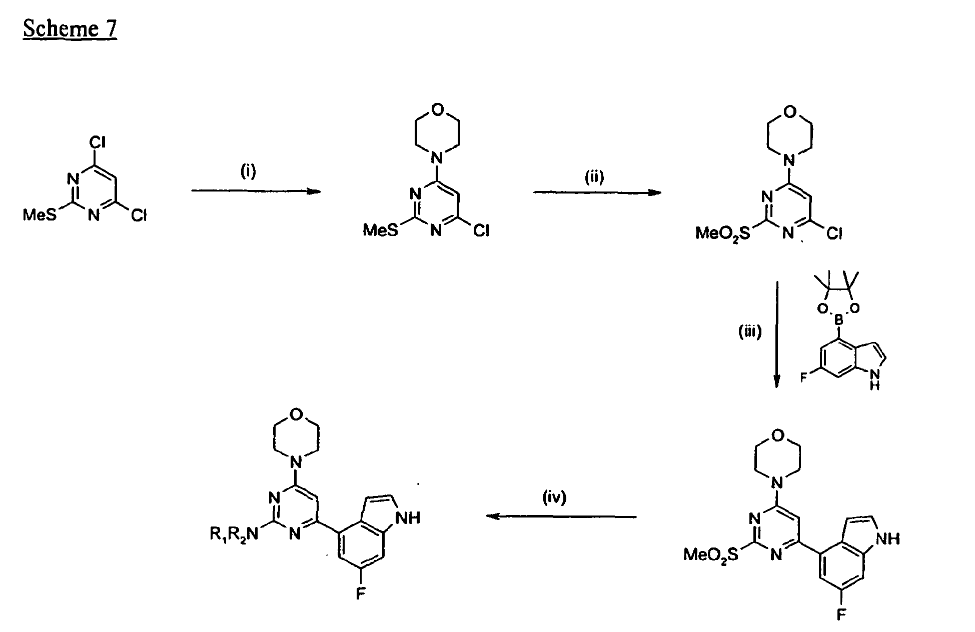 Conditions: (i) morpholine, THF, DIPEA, 60°C, 18 h. (ii) Oxone®, Bu₄NHSO₄, DCM, RT, 18 h. (iii) boronate ester, PdCl₂(PCy₃)₂, K₃PO₄, dioxane, microwave, 125°C, 10 minutes. (iv) R1R2NH, dioxan, microwave, 150°C, 1 hour. 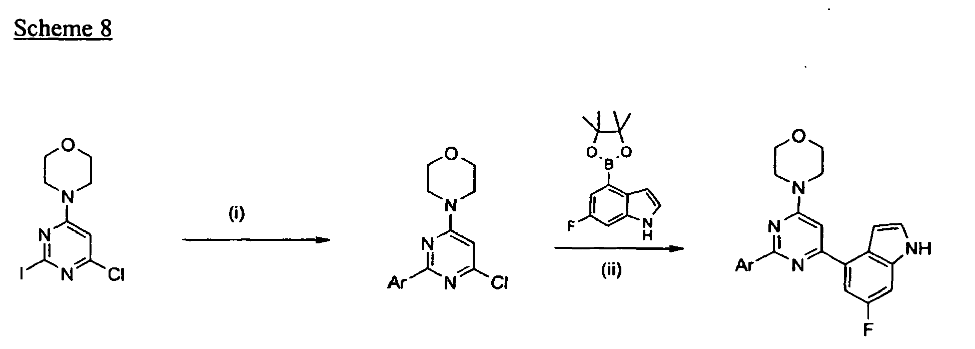 Conditions: (i) ArB(OH)₂, PdCl₂(PPh₃)₂, CH₃CN-H₂O, Na₂CO₃, microwave, 140°C, 45 minutes. (ii) indole boronate ester, PdCl₂(PCy₃)₂, K₃PO₄, dioxane, microwave, 125°C, 15 minutes. 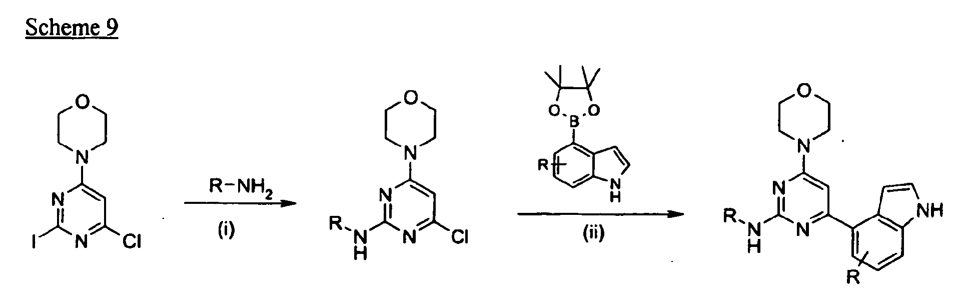 Conditions: (i) R = aliphatic: CuI, K₃PO₄, ethylene glycol, i-propanol, 80 °C, 18 h; R = aromatic: Pd₂(dba)₃, NaO^{t}Bu, DPPF, toluene, RT 16 h or Pd₂(dba)₃, NaO'Bu, Xantphos, dioxane, RT, 4 days. (ii) Suzuki conditions.

### General Experimental Details:

### NMR Spectrometry

NMR spectra were obtained on a Varian Unity Inova 400 spectrometer with a 5 mm inverse detection triple resonance probe operating at 400 MHz or on a Bruker Avance DRX 400 spectrometer with a 5 mm inverse detection triple resonance TXI probe operating at 400 MHz or on a Bruker Avance DPX 400 spectrometer with a 5 mm ¹H/¹³C Dual autotune probe operating at 400 MHz for ¹H or on a Bruker Avance DPX 300 spectrometer with a standard 5mm dual frequency probe operating at 300 MHz. Shifts are given in ppm relative to tetramethylsilane @ 303K.

### Purification by column chromatography:

Compounds purified by column chromatography were purified using silica gel or Isolute® cartridge or Redisep® cartridge, eluting with gradients from 100-0 to 0-100 % of cyclohexane/EtOAc, or from 100-0 to 0-100 % pentane/EtOAc or from 100-0 to 70-30 % DCM/MeOH (with or without the addition of NH₃ 0.1 %). 'Silica gel' refers to silica gel for chromatography, 0.035 to 0.070 mm (220 to 440 mesh) (e.g. Fluka silica gel 60), and an applied pressure of nitrogen up to 10 p.s.i accelerated column elution. Where thin layer chromatography (TLC) has been used, it refers to silica gel TLC using plates, typically 3 × 6 cm silica gel on aluminium foil plates with a fluorescent indicator (254 nm), (e.g. Fluka 60778).

### Purification by preparative HPLC:

Compounds purified by preparative HPLC were purified using either conditions A: Waters XBridge Prep Phenyl column (150 × 19 mm i.d. column with 5 µm particle size, PDA/MS detection, flow 21.25 ml/min), eluting with gradients from 95-5 % to 5-95 % water/acetonitrile containing 0.1 % dimethylethylamine; or conditions B: C18-reverse-phase column (100 × 22.5 mm i.d. Genesis column with 7 µm particle size, or a Phenyl-Hexyl column (250 x 21.2 mm i.d. Gemini column with 5 µm particle size, UV detection at 230 or 254 nm, flow 5-20 mL/min), UV detection at 230 or 254 nm, flow 5-15 mL/min), eluting with gradients from 100-0 % to 0-100 % water/acetonitrile or water/MeOH containing 0.1 % TFA or water/acetonitrile containing 0.1 % formic acid.. When using conditions B the free base was liberated by partitioning between EtOAc and a sat. solution of sodium bicarbonate. The organic layer was dried (MgSO₄) and concentrated *in vacuo.* Alternatively, the free base was liberated by passing through an Isolute® SCX-2 cartridge, eluting with NH₃ in methanol.

### General method used for BOC-deprotection:

To a solution of BOC-amino-pyrimidine in DCM was added TFA and the resulting solution was stirred at RT for 30-180 min. The resulting mixture was diluted with water then extracted with DCM. The combined organic extracts were dried (MgSO₄ or Na₂SO₄), filtered and concentrated *in vacuo,* then purified by either preparative HPLC or column chromatography to give the desired product. Alternatively, the reaction mixture was loaded onto an Isolute® SCX-2 cartridge, washed with MeOH then eluted with 2 M NH₃ in MeOH. The resulting residue was then purified by either preparative HPLC or column chromatography to give the desired product.

### Abbreviations used in the experimental section:

aq. = aqueous
BOC = t-Butoxycarbonyl
bs = broad singlet (NMR)
Cs₂CO₃ = cesium carbonate
d = doublet (NMR)
DCM = dichloromethane
DIPEA = diisopropylethylamine
DMA = dimethylacetamide
DMAP = dimethylaminopyridine
DME = dimethoxyethane
DMF = dimethylformamide
DMSO = dimethylsulfoxide
eq. = equivalents
EtOAc = ethyl acetate
EtOH = ethanol
h = hour(s)
HATU = *O*-(7-Azabenzotriazol-1-yl)-*N,N,N',N'*-tetramethyluronium hexafluorophosphate
HCl = hydrochloric acid
H₂O = water
HPLC = high pressure liquid chromatography
IMS = industrial methylated spirit
iPrOH = isopropanol
LCMS = liquid chromatography mass spectrometry
M = molar
m = multiplet (NMR)
MeOH = methanol
mg = milligram
MgSO₄ = magnesium sulphate
min = minute(s)
mL = millilitre
Na₂CO₃ = sodium carbonate
NaHCO₃ = sodium hydrogen carbonate
NaOH = sodium hydroxide
Na₂SO₄ = sodium sulfate
NMR = nuclear magnetic resonance
q = quartet (NMR)
Rt = retention time
RT = room temperature
sat = saturated
t = triplet (NMR)
TFA = trifluoroacetic acid
THF = tetrahydrofuran
TLC = thin layer chromatography

### Reference Example 1: Formation of boronate ester

The boronate ester product of the final step of scheme 1 above was prepared as follows. To a solution of halide (1 eq.) and bis(pinacolato)diboron (1.3 eq.) in DMSO were added KOAc (3 eq.) and [1,1'-bis(diphenylphosphine)ferrocene]-dichloropalladium (0.05 eq.). The mixture was heated at 90 °C until completion of the reaction. The reaction mixture was partioned between EtOAc and H₂O. The organic layer was washed successively with H₂O and brine, dried over Na₂SO₄ and evaporated to dryness. The resultant residue was then purified by column chromatography.

### Reference Example 2: Suzuki coupling

The Suzuki coupling depicted in general terms in scheme 3 above was performed using one of the following three synthetic strategies:

### Method A.

A mixture of 2-chloro-pyrimidine (1 eq.), Cs₂CO₃ (1.5 eq.), indole boronate ester (1.2 eq.) and tetrakis(triphenylphosphine)palladium (0.05 eq.) in dioxane/water (3:1) was heated at 125 °C, for 10 - 30 min in a microwave reactor (Smith synthetiser). The resulting mixture was diluted with water then extracted with ethyl acetate. The combined organic layers were dried (MgSO₄), filtered and concentrated then purified by either preparative HPLC or column chromatography to give the desired product.

### Method B.

A stirred mixture of chloropyrimidine (1 eq.), indole boronate ester (1.4 eq.) and PdCl₂(PCy₃)₂ (0.02 eq.) in K₃PO₄ (0.5 ml of a 1.27 M aqueous solution) and dioxane (1.0 mL) was heated at 125 °C in a microwave for 15 min. The product was purified by catch-and-release using an Isolute SCX-2 cartridge followed by flash chromatography to give the desired product

### Method C.

A mixture of chloropyrimidine (1 eq.), 1 M aqueous Na₂CO₃ (3 eq.), indole boronate ester (1.8 eq) and dichlorobis(triphenylphosphine) palladium (II) (0.05 eq.) in acetonitrile was heated for 30 - 60 minutes in a microwave reactor at 140C. The mixture was partitioned between water and dichloromethane, the combined organic layers washed with brine, separated and dried (MgSO₄). The crude product was purified by column chromatography to give the desired compound.

### Reference Example 3 4-N,N-Trimethyl-3-nitro-benzenesulfonamide

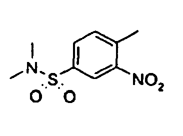

To a solution of dimethylamine in H₂O (40% w/w, 15.0 mL, 120 mmol) at 0 °C was added a solution of 4-methyl-3-nitro-benzenesulfonyl chloride (9.42 g, 40 mmol) in DCM (60 mL) over 30 min. The resulting mixture was stirred at 0 °C for 30 min before being allowed to warm to RT and stirred overnight. The reaction mixture was diluted with H₂O (100 mL) and DCM (40 mL), and the layers were separated. The organic layer was washed in succession with water, HCl (aq., 0.1 M) and brine before being dried over Na₂SO₄ and evaporated to dryness to give the title compound as a pale yellow solid (9.13 g, 94 %).
[M + H]⁺ 244.9

### Reference Example 4: 3-Bromo-4-N,N-trimethyl-5-nitro-benzenesulfonamide

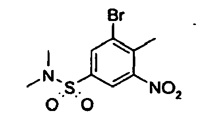

To a solution of 4-*N,N*-trimethyl-3-nitro-benzenesulfonamide (8.57 g, 34.7 mmol) in concentrated sulfuric acid (80 mL) was added 1,3-dibromo-[1,3,5]triazinane-2,4,6-trione (5.97 g, 20.8 mmol) and the orange reaction mixture was stirred at RT for 16 h. A further 2 g of 1,3-dibromo-[1,3,5]triazinane-2,4,6-trione was added and stirring continued for 5 h. The reaction mixture was then poured onto ice and water and stirred for 15 min. The resulting milky/white solid was filtered and washed with H₂O, before being dissolved in EtOAc. The organic layer was dried over Na₂SO₄ and evaporated to dryness to give the title compound as a white solid (10.41 g, 93 %).
[M + H]⁺ 323.1 (⁷⁹Br) 325.0 (⁸¹Br)

### Reference Example 5: 1-Bromo-5-methanesulfonyl-2-methyl-3-nitro-benzene

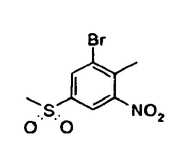

Prepared according to the method used in the preparation of 3-bromo-4-*N,N*-trimethyl-5-nitro-benzenesulfonamide using 4-methanesulfonyl-1-methyl-2-nitro-benzene in place of 4-*N,N*-trimethyl-3-nitro-benzenesulfonamide. The title compound was obtained as a white solid (17.0 g, 85 %).
[M + H]⁺ 294.1 (⁷⁹Br) 296.0 (⁸¹Br)

### Reference Example 6: 1-Bromo-5-fluoro-2-methyl-3-nitro-benzene

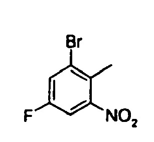

Prepared according to the method used in the preparation of 3-bromo-4-*N,N*-trimethyl-5-nitro-benzenesulfonamide using 4-fluoro-1-methyl-2-nitro-benzene in place of 4-*N,N-*trimethyl-3-nitro-benzenesulfonamide. The title compound was obtained as a yellow solid (68.0 g, 79 %).
NMR δ_{H} (300 MHz, CDCl₃) 2.59 (s, 3H), 7.50 (dd, J = 2.8, 7.6, 1H) and 7.58 (dd, J = 2.9, 7.4, 1H).

### Reference Example 7 4-Bromo-1H-indole-6-sulfonic acid dimethylamide

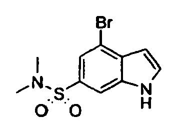

To a solution of 3-bromo-4-*N,N*-trimethyl-5-nitro-benzenesulfonamide (9.15 g, 28.3 mmol) in dioxane (60 mL) was added DMF-DMA (11.3 mL, 84.9 mmol). The deep red reaction mixture was heated at 80°C for 24 h followed by heating at 90°C for 16 h. The mixture was cooled to RT and concentrated to 50 % of the volume, poured into H₂O and extracted into EtOAc. The organic layer was isolated and washed with H₂O, then brine, dried over Na₂SO₄, and evaporated to dryness to give 3-bromo-4-(2-dimethylamino-vinyl)-*N,N-*dimethyl-5-nitro-benzenesulfonamide as a red solid (10.4 g, 91 %). To a suspension of the amide (10.4 g, 25.7 mmol) and Raney®-Nickel (suspension in H₂O, 20 mL) in MeOH:THF (1:1, 200 mL) was added hydrazine monohydrate (1.9 mL, 38.6 mmol) at 0°C and the mixture stirred at RT for 40 min. The reaction mixture was then filtered through Celite and the filter cake washed with EtOAc and H₂O. The aqueous layer was isolated and then extracted with EtOAc. The combined organic layers were washed with H₂O, followed by brine, dried over Na₂SO₄ then evaporated to dryness. The resulting pink solid was purified by column chromatography, and subsequently recrystallised from iPrOH and EtOH to give the title compound as a white solid (3.5 g, 41 %).
NMR δ_{H} (400 MHz, CDCl₃) 2.72 (s, 6H), 6.70 (m, 1H), 7.49 (apparent t, J = 2.7, 1H), 7.68 (d, J = 1.1, 1H), 7.94 (m, 1H) and 9.04 (bs, 1H).

### Reference Example 8 4-Bromo-6-methanesulfonyl-1H-indole

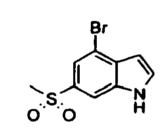

Prepared according to the method used in the preparation of 4-bromo-1*H*-indole-6-sulfonic acid dimethylamide using 1-bromo-5-methanesulfonyl-2-methyl-3-nitro-benzene in place of 3-bromo-4-*N,N-*trimethyl-5-nitro-benzenesulfonamide. The title compound was obtained as a white solid (1.8 g, 76 %).
NMR δ_{H} (300 MHz, CDCl₃) 3.11 (s, 3H), 6.70 (m, 1H), 7.52 (dd, J = 2.5, 3.0, 1H), 7.81 (d, J = 1.5, 1H), 8.10 (dd, J = 1.0, 1.5, 1H) and 9.34 (bs, 1H).

### Reference Example 9 4-Bromo-6-fluoro-1H-indole

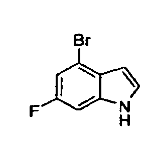

Prepared according to the method used in the preparation of 4-bromo-1*H*-indole-6-sulfonic acid dimethylamide using 1-bromo-5-fluoro-2-methyl-3-nitro-benzene in place of 3-bromo-4-*N,N*-trimethyl-5-nitro-benzenesulfonamide. The title compound was obtained as a white solid (6.06 g, 33 %).
NMR δ_{H} (300 MHz, CDCl₃) 6.57 (apparent t, J = 2.7, 1H), 7.04 (dd, J = 2.1, 9.1, 1H), 7.12 (dd, J = 2.1, 9.1, 1H), 7.20-7.25 (m, 1H) and 8.25 (s, 1H).

### Reference Example 10 2-Methyl-1,3-dinitro-5-trifluoromethyl-benzene

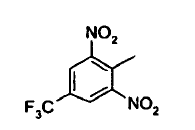

To a solution of 4-methylbenzo-trifluoride (9.51 g, 59.4 mmol) in concentrated sulphuric acid (120 mL) was added potassium nitrate (15.0 g, 0.149 mol) and the resulting mixture stirred at RT for 16 h. The reaction mixture was poured onto ice and water then extracted into EtOAc. The organic layer was washed successively with H₂O and brine, dried over Na₂SO₄ and evaporated to dryness to give the title compound as a yellow solid (13.84 g, 93 %)
NMR δ_{H} (400 MHz, CDCl₃) 2.67 (s, 3H) and 8.27 (s, 2H).

### Reference Example 11 6-Trifluoromethyl-1H-indol-4-ylamine

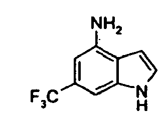

Prepared according to the method used in the preparation of 4-bromo-1*H*-indole-6-sulfonic acid dimethylamide using 2-methyl-1,3-dinitro-5-trifluoromethyl-benzene in place of 3-bromo-4,*N,N*-trimethyl-5-nitro-benzenesulfonamide. The title compound was obtained as a white solid (10.7 g, 99%).
[M + H]⁺ 201.1

### Reference Example 12 4-Iodo-6-trifluoromethyl-1H-indole

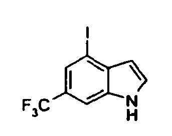

To a suspension of 6-trifluoromethyl-1*H*-indol-4-ylamine (10.7 g, 53.4 mmol) in HCl (aq., 15 %, 240 mL) was added a solution of sodium nitrite (5.52 g, 80.1 mmol) in H₂O (10 mL) slowly at 0 °C. The reaction mixture was stirred at RT for 1 h before a solution of sodium tetrafluoroborate (23.5 g, 0.214 mol) in H₂O (30 mL) was added. After stirring for 15 min, the resulting precipitate was collected by filtration and washed with a sodium tetrafluoroborate solution (aq., sat) before dissolving in acetonitrile (100 mL). This solution was added slowly to a suspension of sodium iodide (24.0 g, 0.160 mol) in acetonitrile (100 mL) and the mixture stirred at RT for 16 h. The reaction mixture was concentrated to 30 % of the volume and partioned between EtOAc and H₂O. The organic layer was isolated then washed in succession with sodium thiosulfate, H₂O and brine, dried over Na₂SO₄ and evaporated to dryness. The resulting brown oil was purified by column chromatography to give the title compound (9.77 g, 59 %).
[M - H]⁻ 310.1

### Reference Example 13 4-(4,4,5,5-Tetramethyl-[1,3,2]dioxaborolan-2-yl)-1H-indole-6-carboxylic acid amide

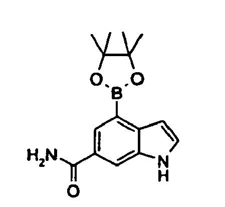

A solution of 4-bromo-1*H*-indole-6-carbonitrile (1 g, 4.50 mmol) in methanol (10 mL) was treated with 30% aqueous hydrogen peroxide (2.7 mL, 4.95 mmol) and a 1. M aqueous sodium hydroxide solution (5 mL) then heated at 40 °C for 1 h. The reaction mixture was cooled, treated with water and cooled in an ice-bath. The resulting precipitate was collected by filtration, washed with water and dried *in vacuo* to obtain 4-bromo-1*H*-indole-6-carboxylic acid amide (1.05 g, 97%), which was transformed into the title boronic ester by the general method (Scheme 1) (0.80 g, 67%).
NMR δ_{H} (300 MHz, DMSO-d₆) 1.35 (s, 12H), 6.78 (m, 1H), 7.10 (s, 1H), 7.51-7.54 (m, 1H), 7.94-7.97 (m, 2H), 8.06 (s, 1H) and 11.40 (bs, 1H).

### Reference Example 14 5-Fluoro-4-(4,4,5,5-tetramethyl-[1,3,2]dioxaborolan-2-yl)-1H-indole

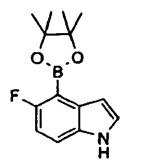

A solution of 5-fluoroindole (5 g, 37.0 mmol) in DMF (40 mL) was treated at 0 °C with trifluoroacetic anhydride (6.1 mL, 42.6 mmol). After 30 min, the reaction was poured into water and the resulting precipitate collected by filtration, washed with water, then dried *in vacuo.* The solid was then dissolved in 10% aqueous NaOH (200 mL) and heated at reflux for 1 h. The reaction mixture was then cooled, washed with dichloromethane and acidified with aqueous HCl. The resulting white precipitate was collected by filtration, washed with water, taken up in dichloromethane, washed with water, dried (MgSO₄) and evaporated *in vacuo.* The resulting material (5 g, 75%) was dissolved in methanol (80 mL) and treated with concentrated sulphuric acid (2 mL) then heated at reflux overnight. The reaction was cooed and the resulting precipitate collected, washed with water and evaporated *in vacuo* to give 5-fluoro-1*H*-indole-3-carboxylic acid methyl ester as a peach-coloured solid (4.5 g, 83 %).

A solution of thallium tris(trifluoroacetate) (8.45 g, 15.6 mmol) in TFA (35 mL) was added to a solution of 5-fluoro-1*H*-indole-3-carboxylic acid methyl ester (2 g, 10.4 mmol) in TFA (10 mL) at room temperature and stirred for 2 h. The reaction mixture was evaporated *in vacuo* and the resulting residue suspended in water (25 mL) before being treated with a solution of potassium iodide (5.2 g, 31.3 mmol) in water (50 mL). The reaction mixture was treated with dichloromethane (100 mL) and methanol (5 mL) and the resulting precipitate removed by filtration through celite. The organic layer was separated, washed successively with sodium thiosulfate solution and brine, then dried (MgSO₄) and evaporated *in vacuo.* The resultant material was dissolved in methanol (60 mL) and treated with 40% aqueous NaOH solution (60 mL) then refluxed for 2 h. The reaction mixture was cooled and extracted with DCM/MeOH (ratio 95:5), dried (MgSO₄), filtered and evaporated *in vacuo* to give a crude solid. Purification by column chromatography gave 5-fluoro-4-iodo-1*H*-indole as a pale brown solid (1.05 g, 39 %).
NMR δ_{H} (300 MHz, CDCl₃) 6.49-6.52 (m, 1H), 6.95 (apparent dt, J = 0.4, 8.6, 1H), 7.26-7.33 (m, 2H) and 8.35 (s, 1H).

A solution of 5-fluoro-4-iodo-1*H*-indole (261 mg, 1.0 mmol) in dioxane (1 mL) was treated with triethylamine (0.2 mL, 1.4 mmol), palladium acetate (4.5 mg, 0.02 mmol) and bis(cyclohexyl)phosphino-2-biphenyl (28 mg, 0.08 mmol) then heated to 80°C. A solution of pinacolborane (1 M in THF, 2.66 mL, 2.66 mmol) was added via syringe. After 30 min, the reaction mixture was cooled, then diluted with water (10 mL) and DCM (10 mL). The resulting mixture was passed through a phase separation cartridge, and the dichloromethane layer was evaporated *in vacuo* to obtain the title compound which was used without further purification.

### Reference Example 15 4-Chloro-6-morpholin-4-yl-pyrimidine-2-carbonitrile

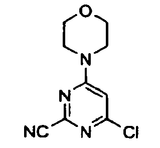

To a mixture of 4-(6-chloro-2-iodo-pyrimidin-4-yl)-morpholine (2 g, 6.14 mmol), tris-(dibenzylideneacetone)dipalladium (113 mg, 0.123 mmol), 1,1'-bis(diphenylphosphino)-ferrocene (136 mg, 0.246 mmol), zinc dust (48 mg, 0.737 mmol) and zinc cyanide (433 mg, 3.69 mmol) was added DMA (20 mL). The reaction mixture was degassed then heated at 120°C for 45 min. The reaction mixture was left to cool to RT and diluted with EtOAc and aqueous ammonia (33 %). The organic layer was isolated, washed with brine then dried (Na₂SO₄) and concentrated *in vacuo.* The resultant residue was purified by column chromatography to afford the title compound as a yellow solid (910 mg, 66 %).
[M]⁺ 224.6

### Reference Example 16 4-Chloro-6-morpholin-4-yl-pyrimidine-2-carboxylic acid methyl ester

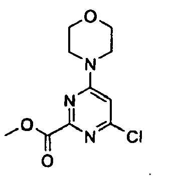

4-Chloro-6-moipholin-4-yl-pyrimidine-2-carbonitrile (1 g, 4.46 mmol) was dissolved in a sat. solution of HCl in MeOH (40 mL) and heated at reflux for 3 h. The solvent was evaporated and the resultant residue was dissolved in DCM, then washed with an aqueous sat. solution of NaHCO₃. The organic layer was dried (MgSO₄) and concentrated *in vacuo* to yield the title compound as a yellow solid (851 mg, 74 %).
[M]⁺ 257.7

### Reference Example 17 4-Chloro-6-morpholin4-yl-pyrimidine-2-carboxylic acid (2-dimethylamino-ethyl)-amide

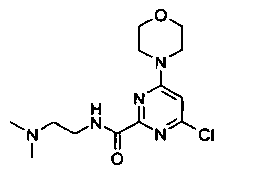

To a solution of *N,N*-dimethylethyleneamine (128 µL, 1.16 mmol) in anhydrous toluene (8 mL) was added trimethylaluminium (2 M in toluene, 0.58 mL, 1.16 mmol) at 0°C. The resulting mixture was stirred at 0°C for 30 min. 4-Chloro-6-morpholin-4-yl-pyrimidine-2-carboxylic acid methyl ester (300 mg, 1.16 mmol) was added portionwise at 0°C. The reaction mixture was allowed to warm to RT and stirred for 16 h. A solution of NaOH (aq., 4 M) was added drop-wise and the aqueous layer was extracted with EtOAc. The combined organic layers were washed with brine, dried (MgSO₄), then concentrated *in vacuo.* The resultant residue was purified by column chromatography to give the title compound as a tan solid (200 mg, 55 %).
[M + H]⁺ 314.2

### Reference Example 18 4-Chloro-6-morpholin-4-yl-pyrimidine-2-carboxylic acid pyridin-3-ylamide

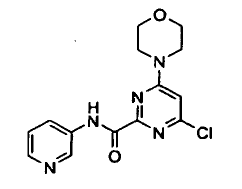

Prepared according to the method used in the preparation of 4-chloro-6-morpholin-4-yl-pyrimidine-2-carboxylic acid (2-dimethylamino-ethyl)-amide using 3-aminopyridine in place of *N,N*-dimethylethyleneamine. The title compound was obtained as a white solid (55.2 mg, 59 %).
[M + H]⁺ 320.2

### Reference Example 19 (4-Chloro-6-morpholin4-yl-pyrimidin-2-yl)-(2-pyridin-3-yl-ethyl)-amine

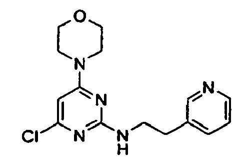

4-(2,6-Dichloro-pyrimidin-4-yl)-morpholine was prepared as described in WO2006/060194.

To a stirred solution of 4-(2,6-dichloro-pyrimidin-4-yl)-morpholine (936 mg; 4.0 mmol) and DIPEA (0.77 ml; 4.4 mmol) in dioxane (10 ml) at 5°C was added 3-(2-aminoethyl)pyridine (0.53 ml; 4.4 mmol). The cooling bath was removed and the reaction mixture stirred at r.t. for 5 h then heated at 70°C for 40 h. Volatiles were removed in vacuo and the residue purified by flash chromatography (95:5 EtOAc/MeOH as eluent) to afford the title compound as a white crystalline solid (963 mg; 75 %).
δ_{H} (400 MHz, CDCl₃) 2.91 (t, J = 7.2, 2H), 3.57 (t, J = 4.8, 4H), 3.66 (q, J = 7.2, 2H), 3.77 (t, J = 4.8, 4H), 5.00 (br s, 1H), 5.90 (s, 1H), 7.24 (dd, J = 7.6 and 4.8, 1H), 7.55 (d, J = 7.6, 1H), 8.49-8.51 (m, 2H).

### Reference Example 20 4-(6-Chloro-2-iodo-pyrimidin-4-yl)-morpholine

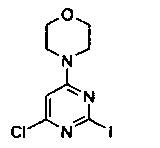

4-Chloro-6-morpholin-4-yl-pyrimidin-2-ylamine was prepared as described in Acta Crystallogr. Sect. C: Cryst. Struct. Commun.; EN; 59; 1; 2003; 4 - 8.

To a mixture of 4-chloro-6-morpholin-4-yl-pyrimidin-2-ylamine (200 mg, 0.93 mmol), diiodomethane (0.37 mL, 4.59 mmol) and copper(I)iodide (177 mg, 0.93 mmol) in tetrahydrofuran (5 mL) was added isoamyl nitrite (0.36 mL, 2.75 mmol). The mixture was flushed out with nitrogen and heated to reflux for 1 hour. The cooled reaction mixture was partitioned between ethyl acetate and 1M hydrochloric acid. The organic layers were washed with concentrated aqueous ammonia followed by saturated aqueous ammonium chloride and dried (MgSO₄). The crude product was purified by column chromatography to give the title compound as a yellow solid (141mg).
δ_{H} (400 MHz, CDCl₃) 3.63 (br m, 4H), 3.78 (t, J = 4.9, 4H), 6.44 (s, 1H).
[M + H]⁺ 325.95

### Reference Example 21 6-Fluoro-4-(2-methanesulfonyl-6-morpholin-4-yl-pyrimidin-4-yl)-1H-indole

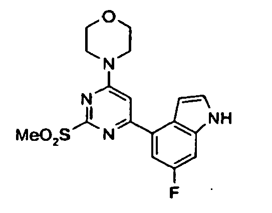

To a stirred solution of 4,6-dichloro-2-(methylthio)pyrimidine (15.44 g; 79 mmol) and DIPEA (15 ml; 86 mmol) in THF (200 ml) at RT. was added morpholine (7.6 mL; 87 mmol) in one portion (a thick white precipitate forms within a few minutes). The reaction mixture was heated at 60°C overnight (18 h) during which time all solids dissolve. The cooled reaction mixture was poured into well-stirred water (1.5 L) and the resulting white solid was collected by filtration, washed with water and dried to afford 4-(6-chloro-2-methylsulfanyl-pyrimidin-4-yl)-morpholine as a white solid (19.03 g; 98 %).

To a stirred solution of Oxone® (30.74 g; 50 mmol) and Bu₄NHSO₄ (0.68 g; 2.0 mmol) was added a solution of 4-(6-chloro-2-methylsulfanyi-pyrimidin-4-yl)-morpholine (4.91 g; 20 mmol) in CH₂Cl₂ (150 mL). The biphasic mixture was stirred vigorously overnight (18 h) upon which time the remaining CH₂Cl₂ was removed *in vacuo.* The resulting precipitate was collected by filtration, washed with water and dried to afford 4-(6-chloro-2-methanesulfonyl-pyrimidin-4-yl)-morpholine as a white solid (4.37 g; 79 %)
[M+H]⁺ 278.

A stirred mixture of 4-(6-chloro-2-methanesulfonyl-pyrimidin-4-yl)-morpholine (1.0 g; 3.6 mmol), 6-fluoro-4-(4,4,5,5-tetramethyl-[1,3,2]dioxaborolan-2-yl)-1H-indole (1.13 g; 4.3 mmol), PdCl₂(PCy₃)₂ (53 mg; 0.072 mmol), K₃PO₄ (5 ml of a 1.27M aqeuous solution; 6.4 mmol) and dioxane (10 ml) was heated at 125°C in a microwave for 10 min. The reaction mixture was diluted with satd. NaHCO₃ (50 ml) and extracted with CHCl3 (2 x 100 ml). The combined organic layers were dried (Na₂SO₄), concentrated and purified by flash chromatography (70:30 CH₂Cl₂/EtOAc as eluent) to obtain a green solid. Trituration with methanol and drying afforded the title compound as a pale green solid (724 mg; 53 %).
δ_{H} (400 MHz, CDCl₃) 3.40 (s, 3H), 3.83-3.86 (m, 8H), 7.04-7.05 (m, 1H), 7.07 (s, 1H), 7.24-7.27 (m, 1H), 7.34-7.36 (m, 1H), 7.43 (dd, J = 10.4 and 2.4, 1H), 8.40 (br s, 1H).

### Reference Example 22 4-(4,4,5,5-Tetramethyl-[1,3,2]dioxaborolan-2-yl)-1H-indole-6-carbonitrile

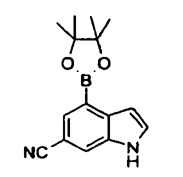

Prepared by using the general method of Scheme 1. The title ompound was obtained as an off-white solid.
δH (400 MHz, CDCl₃) 1.40 (s, 12H), 7.12 (m, 1H), 7.46 (t, J = 2.9, 1H), 7.8 (t, J = 1.1, 1H), 7.87 (d, J = 1.3, 1H), 8.42 (br s, 1H).

### Reference Example 23 4-(4,4,5,5-Tetramethyl-[1,3,2]dioxaborolan-2-yl)-1H-indole-6-sulfonic acid dimethylamide

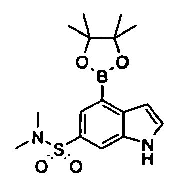

Prepared by using the general method of Scheme 1. The title compound was obtained as a white solid (1.85 g, 46 %).
[M + H]⁺ 350.2 (¹⁰B) 351.2 (¹¹B)

### Reference Example 24 4-(4,4,5,5-Tetramethyl-[1,3,2]dioxaborolan-2-yl)-6-trifluoromethyl-1H-indole

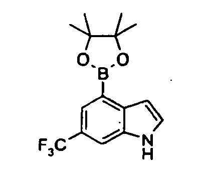

Prepared using the general method of Scheme 1. The title compound was obtained as a pale yellow solid (1.37 g, 92 %).
[M + H]⁺ 311.2 (¹⁰B) 312.2 (¹¹B)

### Reference Example 25 6-Methanesulfonyl-4-(4,4,5,5-tetramethyl-[1,3,2]dioxaborolan-2-yl)-1H-indole

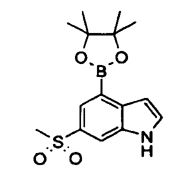

Prepared using the general method of Scheme 1. The title compound was obtained as a pale yellow solid (2.4 g, 51 %).
NMR δ_{H} (300 MHz, DMSO-d₆) 1.36 (s, 12H), 3.18 (s, 3H), 6.87 (m, 1H), 7.73 (apparent t, J = 2.5, 1H), 7.85 (d, J = 1.5, 1H), 8.07 (dd, J = 1.0, 1.5, 1H) and 11.73 (bs, 1H).

### Reference Example 26 6-Fluoro-(4,4,5,5-tetramethyl-[1,3,2]dioxaborolan-2-yl)-1H-indole

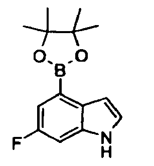

Prepared by using the general method of Scheme 1. The title compound was obtained as a white solid (4.6 g, 61 %).
NMR δ_{H} (300 MHz, CDCl₃) 1.39 (s, 12H), 7.02 (m, 1H), 7.14-7.19 (m, 1H), 7.20-7.26 (m, 1H), 7.38 (dd, J = 2.4, 9.9, 1H) and 8.16 (s, 1H).

### Reference Example 27 7-(4-Chloro-6-morpholin-4-yl-pyrimidin-2-ylamino)-3,4-dihydro-1H-isoquinoline-2-carboxylic acid tert-butyl ester

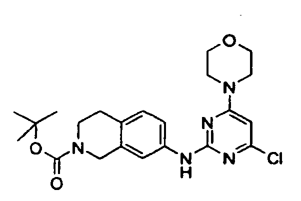

To a mixture of 4-(6-chloro-2-iodo-pyrimidin-4-yl)-morpholine (100 mg, 0.307 mmol) and 7-amino-3,4-dihydro-1*H*-isoquinoline-2-carboxylic acid tert-butyl ester (114 mg, 0.461 mmol) in anhydrous toluene (2 mL) were added tris(dibenzylideneacetone)dipalladium (8 mg, 0.009 mmol), 1,1'-bis(diphenylphosphino)ferrocene (12 mg, 0.021 mmol) and sodium tert-butoxide (74 mg, 0.768 mmol). The reaction mixture was degassed and purged with argon, heated at 30 °C for 20 min then stirred at RT for 16 h. The crude reaction mixture was loaded onto a Isolute® SCX-2 cartridge, washed with MeOH then eluted with 2 M NH₃ in MeOH. The resultant residue was purified by column chromatography to afford the title compound as a brown glass (137 mg, 100 %).
[M + H]⁺ 446.3

### Reference Example 28 6-(4-Chloro-6-morpholin-4-yl-pyrimidin-2-ylamino)-3,4-dihydro-1H-isoquinoline-2-carboxylic acid tert-butyl ester

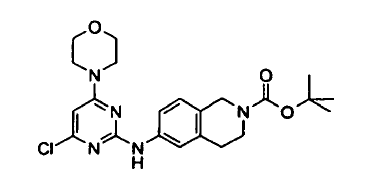

A mixture of 4-(6-chloro-2-iodo-pyrimidin-4-yl)-morpholine (1.12 g; 3.4 mmol), 6-amino-3,4-dihydro-1*H*-isoquinoline-2-carboxylic acid tert-butyl ester (1.88 g; 7.6 mmol), NaOBu*^{t}* (0.46 g; 4.8 mmol), Pd₂dba₃ (47 mg; 0.051 mmol) and Xantphos (98 mg; 0.17 mmol) in dioxane (40 ml) was stirred under argon at r.t. for 4 days. The reaction mixture was diluted with water (50 ml) and brine (150 ml) then extracted into EtOAc (2 × 100 ml). The combined organic layers were dried (Na₂SO₄), concentrated and purified by ISCO chromatography to obtain the title compound as a white solid (543 mg; 36 %).
δ_{H} (400 MHz, CDCl₃) 1.51 (s, 9H), 2.81-2.84 (m, 2H), 3.60-3.67 (m, 6H), 3.78-3.81 (m, 4H), 4.55 (s, 2H), 6.04 (s, 1H), 6.85 (s, 1H), 7.07 (d, J = 8.0, 1H), 7.32 (s, 1H), 7.37 (d, J = 8.0, 1H).

### Reference Example 29 7-Amino-3,4-dihydro-1H-isoquinoline-2-carboxylic acid benzyl ester

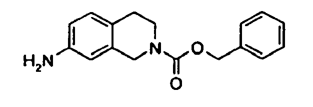

1,2,3,4-Tetrahydroisoquinoline (10 ml; 80 mmol) was added dropwise over 10 min. to stirred conc. H₂SO₄ at 0 °C. KNO₃ was added portionwise over 5 min. at 0 °C and the resulting mixture was stirred overnight whilst allowing to warm to r.t. The reaction mixture was quenched by pouring onto iced water (300 ml) and basified with NH₄OH. The mixture was extracted with CH₂Cl₂ (2 x 300 ml), the combined organic layers were dried (Na₂SO₄) and concentrated. The dark red residue was dissolved in EtOH (400 ml) and treated with HCl_{(g)} for 5 min. The resulting solid was recrystallised from EtOH to give 7-nitro-1,2,3,4-tetrahydro-isoquinoline hydrochloride as an off-white solid (4.69 g).

7-nitro-1,2,3,4-tetrahydro-isoquinoline hydrochloride (2.57 g) was stirred in CH₂Cl₂/satd. NaHCO₃ for 40 min. After separation of the layers (hydrophobic frit) the solvent was evaporated and the resulting solid dissolved in EtOH (80 ml), PtO₂ (112 mg) was added and the mixture was stirred under an atmosphere of hydrogen for 3h. The catalyst was removed by filtration and solvent evaporated to give 7-amino-1,2,3,4-tetrahydro-isoquinoline as a brown solid (1.73 g).

To a stirred solution of 7-amino-1,2,3,4-tetrahydro-isoquinoline (1.73 g; 11.7 mmol) and NEt₃ (1.9 ml; 13.6 mmol) in CH₂Cl₂ (30 ml) at r.t. was added benzyl chloroformate (1.8 ml; 12.5 mmol). The reaction mixture was stirred at r.t. for 3 h upon which time it was quenched with satd. NaHCO₃ (30 ml), the layers were separated (hydrophobic frit) and solvent evaporated. Purification by flash chromatography gave the title compound as an off-white solid (1.85 g; 56 %).
δ_{H} (400 MHz, CDCl₃) 2.76 (br s, 2H), 3.59 (br s, 2H), 3.71 (br s, 2H), 4.58 (s, 2H), 5.20 (s, 2H), 6.43-6.57 (m, 2H), 6.94 (d, J = 7.6, 1H), 7.33-7.42 (m, 5H).

### Reference Example 30 7-(4-Chloro-6-morpholin-4-yl-pyrimidin-2-ylamino)-3,4-dihydro-1H-isoquinoline-2-carboxylic acid benzyl ester

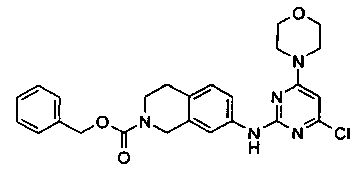

A stirred solution of 7-amino-3,4-dihydro-1*H*-isoquinoline-2-carboxylic acid benzyl ester (0.85 g; 3.0 mmol), 4-(2,6-dichloro-pyrimidin-4-yl)-morpholine (0.59 g; 2.5 mmol) and conc. HCl (7 drops) in dioxane (10 ml) was heated in a microwave at 110 °C for 1 h. The reaction mixture was diluted with CH₂Cl₂ (40 ml) and washed with satd. Na₂CO₃ (40 ml). The organic layer was separated (hydrophobic frit), concentrated and purified by flash chromatography to yield the title compound as an off-white solid (430 mg; 36 %).
δ_{H} (400 MHz, CDCl₃) 2.74 (br s, 2H), 3.52-3.68 (m, 10H), 4.55 (br s, 2H), 5.11 (s, 2H), 5.95 (s, 1H), 6,76 (br s, 1H), 7.00 (d, J = 8.0, 1H), 7.25-7.45 (m, 8H).

### Reference Example 31 5-(4-Chloro-6-morpholin-4-yl-pyrimidin-2-ylamino)-3,4-dihydro-1H-isoquinoline-2-carboxylic acid tert-butyl ester

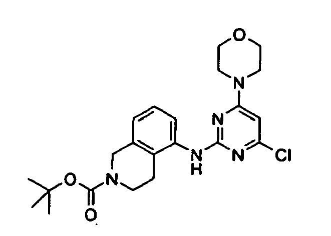

A mixture of 4-(6-chloro-2-iodo-pyrimidin-4-yl)-morpholine (326 mg; 1.0 mmol), 5-amino-3,4-dihydro-1*H*-isoquinoline-2-carboxylic acid tert-butyl ester (323 mg; 1.3 mmol), NaOBu*^{t}* (135 mg; 1.4 mmol), Pd₂dba₃ (13.7 mg; 0.015 mmol) and Xantphos (28.9 mg; 0.05 mmol) in dioxane (2.5 ml) was stirred under argon at 80 °C for 3 h. The reaction mixture was diluted with water (10 ml) and brine (20 ml) then extracted into CH₂Cl₂ (2 x 35 ml). The combined organic layers were dried (Na₂SO₄), concentrated and purified by flash chromatography (60:40 to 70:30 EtOAc/Petrol) to obtain the title compound as a white solid (114 mg).
δ_{H} (400 MHz, CDCl₃) 1.51 (s, 9H), 2.75 (t, J = 6.0, 2H), 3.55-3.58 (m, 4H), 3.69 (t, J = 6.0, 2H), 3.74-3.77 (m, 4H), 4.61 (s, 2H), 6.03 (s, 1H), 6.59 (br s, 1H), 6.92 (d, J = 8.0, 1H), 7.22 (t, J = 8.0, 1H), 7.75 (d, J = 8.0, 1H).

### Reference Example 32 (4-Chloro-6-morpholin-4-yl-pyrimidin-2-yl)-(2-thiazol-2-yl-ethyl)-amine

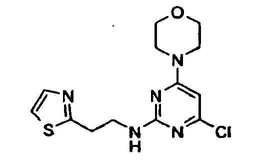

To a mixture of 4-(6-chloro-2-iodo-pyrimidin-4-yl)-morpholine (163 mg, 0.500 mmol) and 2-thiazol-2-yl-ethylamine hydrochloride (95 mg, 0.575 mmol) in anhydrous toluene (2 mL) were added palladium(II) acetate (1 mg, 0.005 mmol), 2,2'-bis(diphenylphosphino)-1,1'-binaphthalene (4.5 mg, 0.007 mmol) and sodium tert-butoxide (134 mg, 1.4 mmol). The reaction mixture was degassed and purged with argon then heated at 80 °C for 16 h. The crude reaction mixture was partitioned between DCM and brine. The organic layer was isolated, dried (MgSO₄) then concentrated *in vacuo.* The resultant residue was purified by column chromatography to give the title compound as a white solid (77 mg, 48 %).
[M + H]⁺ 326.1

### Reference Example 33 2-Bromomethyl-6-methyl-pyridine

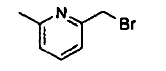

To a solution of (6-methyl-pyridin-2-yl)-methanol (0.6 g, 4.872 mmol) in DCM (18 mL) were added triphenylphosphine (1.68 g, 6.405 mmol) and carbon tetrabromide (1.97 g, 5.940 mmol) before the resulting mixture was stirred at RT for 3 h. The crude reaction mixture was then concentrated *in vacuo* and the resultant residue purified by column chromatography to give the title compound as a pale pink oil (0.47 g, 52 %).
[M + H]⁺ 185.8 (⁷⁹Br) 187.8 (⁸¹Br)

### Reference Example 34 (6-Methyl-pyridin-2-yl)-acetonitrile

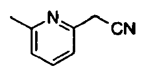

To a solution of 2-bromomethyl-6-methyl-pyridine (0.47 g, 2.526 mmol) in acetonitrile (25 mL) were added trimethylsilyl cyanide (0.5 mL, 3.75 mmol) and TBAF (1 M in THF, 3.8 mL, 3.8 mmol) before the resulting mixture was heated at reflux for 15 min. The mixture was cooled to RT and NH₄0H was carefully added. The mixture was extracted with EtOAc and the layers separated. The organic layer was dried (MgSO₄) then concentrated *in vacuo.* The resultant residue was purified by column chromatography to give the title compound as colourless oil (0.326 mg, 98 %).
¹H NMR (400 MHz, CDCl₃): δ 2.55 (s, 3 H), 3.89 (s, 2 H), 7.12 (d, J = 7.7, 1 H), 7.25 (d, J = 7.7 Hz, 1 H) and 7.62 (apparent t, J = 7.7 Hz, 1 H).

### Reference Example 35 2-(6-Methyl-pyridin-2-yl)-ethylamine

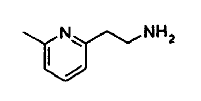

To a solution of (6-methyl-pyridin-2-yl)-acetonitrile (326 mg, 2.467 mmol) in anhydrous THF (60 mL) was added a solution of borane dimethyl sulfide complex in THF (2 M, 5.5 mL, 11.0 mmol) dropwise. The resulting mixture was heated at reflux for 2 h, then cooled to RT. MeOH and a aqueous solution of HCl (1 M, 3 mL) were added and the mixture was stirred at RT for 30 min. The crude reaction mixture was loaded onto a Isolute® SCX-2 cartridge, washed with MeOH then eluted with 2 M NH₃ in MeOH to afford the title compound as a dark yellow oil (188 mg, 56 %).
¹H NMR (400 MHz, CDCl₃): δ 1.35 (bs, 2 H), 2.53 (s, 3 H), 2.89 (t, J = 6.7 Hz, 2 H), 3.08 (t, J = 6.7 Hz, 2 H), 6.98 (m, 2 H) and 7.49 (apparent t, J = 7.65 Hz, 1 H).

### Reference Example 36 2-Fluoro-4-(2-nitro-vinyl)-pyridine

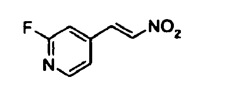

2-Fluoro-pyridine-4-carbaldehyde (270 mg, 2.158 mmol), nitromethane (140 µL, 2.583 mmol) and triethylamine (600 µL, 4.328 mmol) was suspended in DCM (5 mL) and stirred at RT for 1 h, then concentrated *in vacuo* in afford 1-(2-fluoro-pyridin-4-yl)-2-nitro-ethanol as an oil. The crude 1-(2-fluoro-pyridin-4-yl)-2-nitro-ethanol was re-dissolved in DCM (5 mL), then treated with methanesulfonyl chloride (500 µL, 6.46 mmol) and triethylamine (0.94 mL, 6.81 mmol). The reaction mixture was stirred at RT for 20 min, then concentrated *in vacuo.* The resultant residue was purified by column chromatography to give the title compound as a brown solid (198 mg, 55 %).
¹H NMR (400 MHz, CDCl₃): µ 7.05-7.06 (m, 1 H), 7.31 (dt, J = 5.1, 1.6 Hz, 1 H), 7.64 (d, J = 13.8 Hz, 1 H), 7.92 (d, J = 13.8 Hz, 1 H) and 8.36 (d, J = 5.2 Hz, 1 H).

### Reference Example 37 5-Methoxy-2-(2-nitro-vinyl)-pyridine

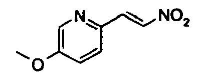

Prepared according to the method used in the preparation of 2-fluoro-4-(2-nitro-vinyl)-pyridine using 5-methoxy-pyridine-2-carbaldehyde in place of 2-fluoro-pyridine-4-carbaldehyde. The title compound was obtained as a brown solid (248 mg, 54 %).
¹H NMR (400 MHz, CDCl₃): δ 3.92 (s, 3 H), 7.21 (dd, J = 8.5, 3.0 Hz, 1 H), 7.43 (d, J = 8.5 Hz, 1 H), 7.90 (s, 2 H) and 8.37 (d, J = 3.0 Hz, 1 H).

### Reference Example 38 2-(2-Fluoro-pyridin-4-yl)-ethylamine

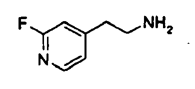

To a solution of 2-fluoro-4-(2-nitro-vinyl)-pyridine (198 mg, 1.178 mmol) in anhydrous THF (10 mL) at 0 °C were added glacial acetic acid (300 µL) and sodium borohydride (160 mg, 4.23 mmol). The resulting suspension was stirred at RT for 17 h, then quenched by addition of water. The crude reaction mixture was passed through a plug of silica and concentrated *in vacuo* to afford 2-fluoro-4-(2-nitro-ethyl)-pyridine as a brown solid (160 mg, 80 %). To a solution of 2-fluoro-4-(2-nitro-ethyl)-pyridine (160 mg, 0.940 mmol) in anhydrous MeOH (10 mL) were added ammonium formate (155 mg, 2.458 mmol) and palladium on carbon (77 mg) and the suspension stirred at RT for 1 h. The crude reaction mixture was filtered through Celite and the filtrate concentrated *in vacuo.* Water and DCM were added to the residue and the resulting suspension was loaded onto a Isolute® SCX-2 cartridge, washed with MeOH then eluted with 2 M NH₃ in MeOH to afford the title compound as a yellow oil (59 mg, 45 %).
¹H NMR (400 MHz, CDCl₃): δ 1.40 (bs, 2 H), 2.80 (t, J = 6.9 Hz, 2 H), 3.03 (t, J = 6.9 Hz, 2 H), 6.81 (s, 1 H), 7.04 (d, J = 5.0 Hz, 1 H) and 8.13 (d, J = 5.0 Hz, 1 H).

### Reference Example 39 2-(5-Methoxy-pyridin-2-yl)-ethylamine

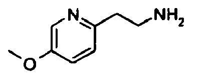

Prepared according to the method used in the preparation of 2-(2-fluoro-pyridin-4-yl)-ethylamine using 5-methoxy-2-(2-nitro-vinyl)-pyridine in place of 2-fluoro-4-(2-nitro-vinyl)-pyridine. The title compound was obtained as a pale yellow solid (220 mg, 81 %).
¹H NMR (400 MHz, CDCl₃): δ 1.43 (s, 1 H), 3.10 (t, J = 5.6 Hz, 2 H), 3.40 (t, J = 5.6 Hz, 2 H), 3.85 (s, 3 H), 7.10 (d, J = 8.5 Hz, 1 H), 7.18 (dd, J = 8.5, 3.0 Hz, 1 H) and 8.14 (d, J = 3.0 Hz, 1 H).

### Reference Example 40 (4-Chloro-6-morpholin-4-yl-pyrimidin-2-yl)-[2-(6-methyl-pyridin-2-yl)-ethyl]-amine

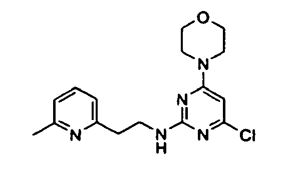

4-(6-Chloro-2-iodo-pyrimidin-4-yl)-moipholine (102 mg, 0.313 mmol), potassium phosphate (132 mg, 0.622 mmol), copper (I) iodide (3.1 mg, 0.016 mmol), 2-(6-methyl-pyridin-2-yl)-ethylamine (51 mg, 0.374 mmol) and ethylene glycol (35 µL, 0.627 mmol) were suspended in 2-propanol (1 mL) under a nitrogen atmosphere and heated at 80 °C for 18 h. The reaction mixture was cooled to RT and diluted with ethyl acetate, then washed with water and brine. The organic layer was isolated, dried (MgSO₄) then concentrated *in vacuo.* The resultant residue was purified by column chromatography to give the title compound as a tan solid (27 mg, 26%).
[M + H]⁺ 334.2

### Reference Example 41 (4-Chloro-6-moroholin-4-yl-pvrimidin-2-yl)-[2-(2-fluoro-pyridin-4-yl)-ethyl]-amine

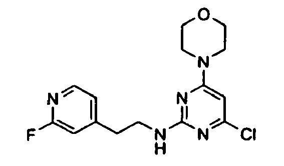

Prepared according to the method used in the preparation of (4-chloro-6-morpholin-4-yl-pyrimidin-2-yl)-[2-(6-methyl-pyridin-2-yl)-ethyl]-amine using 2-(2-fluoro-pyridin-4-yl)-ethylamine in place of 2-(6-methyl-pyridin-2-yl)-ethylamine. The title compound was obtained as a white solid (30.7 mg, 26 %).
[M + H]⁺ 338.1.

### Reference Example 42 (4-Chloro-6-morpholin-4-yl-pyrimidin-2-yl)-[2-(5-methoxy-pyridin-2-yl)-ethyl]-amine

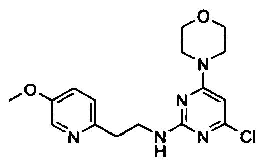

Prepared according to the method used in the preparation of (4-chloro-6-morpholin-4-yl-pyrimidin-2-yl)-[2-(6-methyl-pyridin-2-yl)-ethyl]-amine using 2-(5-methoxy-pyridin-2-yl)-ethylamine in place of 2-(6-methyl-pyridin-2-yl)-ethylamine. The title compound was obtained as a white solid (49.8 mg, 13 %).
[M + H]⁺ 350.1

### Reference Example 43 (4-Chloro-6-morpholin-4-yl-pyrimidin-2-yl)43-pyridin-3-yl-phenyl)-amine

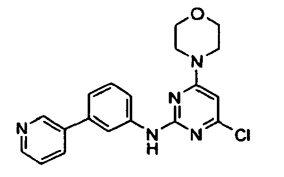

Prepared from 4-(6-chloro-2-iodo-pyrimidin-4-yl)-morpholine and 3-pyridin-3-yl-phenylamine using the method described for 6-(4-chloro-6-morpholin-4-yl-pyrimidin-2-ylamino)-3,4-dihydro-1*H*-isoquinoline-2-carboxylic acid *tert*-butyl ester.
δ_{H} (400 MHz, CDCl₃) 3.64 (m, 4H), 3.79 (m, 4H), 6.08 (s, 1H), 7.09 (s, 1H), 7.25 (m, 1H), 7.38 (m, 1H), 7.44 (t, 1H), 7.51 (m, 1H), 7.88 (dd, 1H), 7.91 (m, 1H), 8.62 (d, 1H), 8.87 (s, 1H).

### Example 1 4-(6-Fluoro-1H-indol-4-yl)-6-morpholin-4-yl-pyrimidine-2-carboxylic acid (2-dimethylamino-ethyl)-amide

Prepared from 4-chloro-6-morpholin-4-yl-pyrimidine-2-carboxylic acid (2-dimethylamino-ethyl)-amide using Suzuki Method A of Reference Example 2. The title compound was obtained as a yellow solid (133 mg, 67 %).
[M + H]⁺ 413.1
¹H NMR (400 MHz, DMSO-d₆): δ 2.21 (s, 6 H), 2.44 (t, J = 6.5 Hz, 2 H), 3.37 (apparent q, J = 6.5 Hz, 2 H), 3.73 (m, 4 H), 3.78 (m, 4 H), 7.15 (m, 1 H), 7.29-7.36 (m, 2 H), 7.48 (t, J = 2.5 Hz, 1 H), 7.66 (dd, J = 11, 2.5 Hz, 1 H), 8.64 (t, J = 5.5 Hz, 1 H) and 11.38 (bs, 1 H).

### Example 2: 4-(6-Fluoro-1H-indol-4-yl)-6-morpholin-4-yl-pyrimidine-2-carboxylic acid pyridin-3-ylamide

Prepared from 4-chloro-6-morpholin-4-yl-pyrimidine-2-carboxylic acid pyridin-3-ylamide using Suzuki Method A of Reference Example 2. The title compound was obtained as a white solid (15 mg, 21 %).
[M + H]⁺ 419.3
¹H NMR (400 MHz, DMSO-d₆): δ 3.76 (m, 4 H), 3.83 (m, 4 H), 7.17 (m, 1 H), 7.36 (m, 1 H), 7.44 (m, 2 H), 7.50 (apparent t, J = 2.5 Hz, 1 H), 7.73 (dd, J = 11, 2.5 Hz, 1 H), 8.24 (m, 1 H), 8.35 (dd, J = 4.5, 1.5 Hz, 1 H), 8.99 (d, J = 2.5 Hz, 1 H), 10.61 (bs, I H) and 11.40 (bs, 1 H).

### Example 3 4-[6-Morpholin-4-yl-2-(2-pyridin-3-yl-ethylamino)-pyrimidin-4-yl]-1H-indole-6-carbonitrile

4-(2-Methanesulfonyl-6-morpholin-4-yl-pyrimidin-4-yl)-1H-indole-6-carbonitrile was prepared as described for 6-fluoro-4-(2-methanesulfonyl-6-morpholin-4-yl-pyrimidin-4-yl)-1H-indole using 4-(4,4,5,5-tetramethyl-[1,3,2]dioxa borolan-2-yl)-1H-indole-6-carbonitrile.

4-(2-methanesulfonyl-6-morpholin-4-yl-pyrimidin-4-yl)-1H-indole-6-carbonitrile (46 mg) and 3-(2-aminoethyl)pyridine (145 µL) in dioxane (1.5 mL) and DIPEA (60 µL) was heated in a microwave reactor for 1 hour at 150 °C. The solvents were evaporated and the residue purified by column chromatography to to give the title compound as a white solid (28 mg).
δ_{H} (400 MHz, CDCl₃) 2.90 (t, J = 6.8, 2H), 3.58-3.60 (m, 4H), 3.67 (q, J = 6.8, 2H), 3.72-3.75 (m, 4H), 5.00 (br s, 1H), 6.26 (s, 1H), 7.01 (s, 1H), 7.15-7.20 (m, 1H), 7.37-7.38 (m, H), 7.49-7.52 (m ,1H), 7.65 (s, 1H), 7.66 (s, 1H), 8.41 (dd, J = 4.8 and 1.6, 1H), 8.45 (d, J = 1.6, 1H), 8.94 (br s, 1H).
[M+H]⁺ 426.

### Example 4: [4-(6-Methanesulfonyl-1H-indol-4-yl)-6-moroholin-4-yl-pyrimidin-2-yl]-(2-pyridin-3-yl-ethyl)-amine

Prepared using Suzuki method B of Reference Example 2. The product was purified by catch-and-release using an Isolute SCX-2 cartridge followed by flash chromatography (94:6:1 CH₂Cl₂/MeOH/NH₄OH as eluent) to afford the title compound as a white solid (63 mg).
δ_{H} (400 MHz, CDCl₃) 3.03 (t, J = 6.8, 2H), 3.13 (s, 3H), 3.69 (t, J = 4.8, 4H), 3.78 (q, J = 6.8, 2H), 3.83 (t, J = 4.8, 4H), 5.04 (br s, 1H), 6.40 (s, 1H), 7.16 (br s, 1H), 7.24-7.28 (m, 1H), 7.52-7.53 (m, 1H), 7.59-7.62 (m, 1H), 8.05 (s, 1H), 8.11 (s, 1H), 8.49-8.52 (m, 1H), 8.56 (d, J = 2.0, 1 H), 8.83 (br s, 1H).
[M+H]⁺: 479.

### Example 5: 4-[6-Moroholin-4-yl-2-(2-pyridin-3-yl-ethylamino)-pyrimidin-4-yl]-1H-indole-6-sulfonic acid dimethylamide

Prepared using Suzuki method B of Reference Example 2. Obtained as a white solid (128 mg).
δ_{H} (400 MHz, CDCl₃) 2.73 (s, 6H), 3.00 (t, J = 6.8), 3.69 (t, J = 4.8, 4H), 3.78 (q, J = 6.8, 2H), 3.83 (t, J = 4.8, 4H), 5.01 (br s, 1H), 6.38 (s, 1H), 7.16 (s, 1H), 7.24-7.27 (m, 1H), 7.50 (br s, 1H), 7.61 (d, J = 7.6), 7.88 (s, 1H), 7.96 (s, 1H), 8.51 (d, J = 4.8), 8.72 (br s, 1H).
[M+H]⁺: 508.

### Example 6: 4-[6-Morpholin-4-yl-2-(2-pyridin-3-yl-ethylamino)-pyrimidin-4-yl]-1H-indole-6-carboxylic acid amide

Prepared using Suzuki method B of Reference Example 2. Obtained as an off-white solid (47 mg).
δ_{H} (400 MHz, CDCl₃) 2.98 (t, J = 6.8, 2H), 3.51 (br s, 4H), 3.77-3.83 (m, 6H), 5.21 (br s, 1H), 5.69 (br s, 1H), 6.43 (s, 1H), 6.98 (s, 1H), 7.21-7.25 (m, 1H), 7.42 (s, 1H), 7.59 (d, J = 7.2, 1H), 7.95 (s, 1H), 8.10 (s, 1H), 8.48 (d, J = 4.0, 1H), 8.60 (s, 1H), 8.94 (br s, 1H).
[M+H]⁺: 444.

### Example 7 [4-Morpholin-4-yl-6-(6-trifluoromethyl-1H-indol-4-yl)-pyrimidin-2-yl]-(2-pyridin-3-yl-ethyl)-amine

Prepared using Suzuki method B of Reference Example 2. Obtained as a yellow solid (72 mg).
δ_{H} (400 MHz, CDCl₃) 3.00 (t, J = 6.8, 2H), 3.69 (t, J = 4.8, 4H), 3.77 (q, J = 6.8, 4H), 3.83 (t, J = 4.8, 4H), 5.05 (br s, 1H), 6.40 (s, 1H), 7.10 (s, 1H), 7.23-7.27 (m, 1H), 7.44 (s, 1H), 7.60 (d, J = 7.6, 1H), 7.75 (s, 1H), 7.79 (s, 1H), 8.50 (d, J = 4.8, 1H), 8.56 (s, 1H).
[M+H]⁺: 469.

### Example 8 [4-(5-Fluoro-1H-indol-4-yl)-6-morpholin-4-yl-pyrimidin-2-yl]-(2-pyridin-3-yl-ethyl)-amine

Prepared using Suzuki method B of Reference Example 2. Obtained as a white solid (70 mg).
δ_{H} (400 MHz, CDCl₃) 2.98 (t, J = 6.8, 2H), 3.66 (t, J = 4.8, 4H), 3.75 (q, J = 6.8, 2H), 3.82 (t, J = 4.8, 4H), 5.00 (br s, 1H), 6.35 (s, 1H), 6.90 (s, 1H), 7.03 (dd, J = 10.8 and 8.8, 1H), 7.23 (dd, J = 7.6 and 4.8, 1H), 7.36 (dd, J = 8.8 and 4.0, 1H), 7.59 (d, J = 7.6, 1H), 8.24 (br s, 1H), 8.49 (d, J = 4.0, 1H), 8.55 (s, 1H).
[M+H]⁺: 419.

### Example 9 6-Fluoro-4-(6-morpholin-4-yl-2-phenyl-pyrimidin-4-yl)-1H-indole

To a solution of 4-(6-chloro-2-iodo-pyrimidin-4-yl)-morpholine (150 mg), phenyl boronic acid (59 mg) and dichlorobis(triphenylphosphine) palladium (II) in acetonitrile (3.5 mL) was added 1M aqueous sodium bicarbonate solution (1.44 mL). The mixture was heated in a microwave reactor for 45 minutes and allowed to cool. The crude mixture was partitioned between dichloromethane and water and the combined organic layers washed with brine, separated and dried (MgSO₄). The crude mixture was purified by chromatography to give 4-(6-chloro-2-phenyl-pyrimidin-4-yl)-morpholine as a white solid (112 mg).

Reaction of 4-(6-chloro-2-phenyl-pyrimidin-4-yl)-morpholine with 6-fluoro-4-(4,4,5,5-tetramethyl-[1,3,2]dioxaborolan-2-yl)-1H-indole using Suzuki Method B gave the title compound as a pale grey solid (168 mg).
δ_{H} (400 MHz, CDCl₃) 3.85 (m, 4H), 3.90 (m, 4H), 6.92 (s, 1H), 7.17 -7.23 (m, 2H), 7.34 (m, 1H), 7.47-7.54 (m, 4H), 8.30 (br s, 1H), 8.58 (m, 1H).
[M + H]⁺ 375.10

### Example 10 6-Fluoro-4-(6-morpholin-4-yl-2-pyridin-3-yl-pyrimidin-4-yl)-1H-indole

4-(6-Chloro-2-pyridin-3-yl-pyrimidin-4-yl)-morpholine was prepared as described for 4-(6-chloro-2-phenyl-pyrimidin-4-yl)-morpholine and then reacted with 6-fluoro-4-(4,4,5,5-tetramethyl-[1,3,2]dioxaborolan-2-yl)-1H-indole using Suzuki Method B of Reference Example 2 to give the title compound as a white solid
(94 mg).
δ_{H} (400 MHz, CDCl₃) 3.77 (m, 4H), 3.81 (m, 4H), 6.87 (s, 1H), 7.07 (m, 1H), 7.14 (dt, J = 1.1, 8.9, 1 H), 7.25 (t, J = 3.3, 1H), 7.33 (m, 1H), 7.41 (dd, J = 2.3, 10.6, 1H), 8.25 (br s, 1H), 8.63 (dd, J = 1.7, 4.8, 1H), 8.71 (dt, J = 2.0, 7.0, 1H), 9.66 (t, J = 1.1, 1H).
[M + H]⁺ 376.12

### Example 11 6-Fluoro-4-(6-morpholin-4-yl-2-pyridin-4-yl-pyrimidin-4-yl)-1H-indole

Prepared by the method described for 6-fluoro-4-(6-morpholin-4-yl-2-pyridin-3-yl-pyrimidin-4-yl)-1H-indole to give the title compound as a white solid (0.034 g).
δ_{H} (400 MHz, CDCl₃) 3.86 (m, 4H), 3.89 (m, 4H), 6.99 (s, I H), 7.15 (s, 1H), 7.25 (m, 1H), 7.36 (m, 1H), 7.51 (m, 1H), 8.39 (t, J = 5.9, 3H), 8.78 (t, J = 5.9, 2H).
[M + H]⁺ 376.1.

### Example 12 6-Fluoro-4-[6-morpholin-4-yl-2-(pyridin-3-yloxy)-pyrimidin-yl]-1H-indole

To a solution of 3-hydroxypyridine (97 mg) in DMF (3 mL) was added sodium hydride (41 mg; 60% dispersion in mineral oil) in a single portion and the mixture stirred at RT for 15 minutes. 4-(2,6-Dichloro-pyrimidin-4-yl)-morpholine (165 mg) was added and the mixture heated at 70°C for 1.5 hours. The mixture was allowed to cool and quenched by the addition of aqueous ammonium chloride and partitioned between ethyl acetate and water. The combined organic layers were washed with brine, separated and dried (MgSO₄). The mixture was purified by preparative HPLC to give 4-(6-chloro-2-pyridin-3-yl-pyrimidin-4-yl)-morpholine compound as a white solid (48 mg).
Suzuki coupling with -(4,4,5,5-tetramethyl-[1,3,2]dioxaborolan-2-yl)-1H-indole using Method B of Reference Example 2 gave the title compound as a pale yellow solid (37 mg). δ_{H} (400 MHz, CDCl₃) 3.59 (t, J = 4.6, 4H), 3.72 (t, J = 4.8, 4H), 6.71 (s, 1H), 6.73 (m, 1H), 7.08 (d, J = 8.8, 1 ), 7.14 (t, J = 2.5, 1H), 7.28 (m, 2H), 7.51 (d, J = 8.3, 1H), 8.21 (br s, 1H), 8.41 (d, J = 4.6, 1H), 8.57 (d, J = 2.4, 1H).
[M + H]⁺ 392.08

### Example 13 [4-(6-Fluoro-1H-indol-4-yl)-6-moroholin-4-yl-pyrimidin-2-yl]-(2-pyridin-3-yl-ethyl)-amine

A mixture of 6-fluoro-4-(2-methanesulfonyl-6-morpholin-4-yl-pyrimidin-4-yl)-1H-indole, (0.085 g), and 3-(2-aminoethyl)pyridine (0.27 g), in 1,4-dioxan (2.0 mL), was heated in a microwave reactor for I hour at 150°C. The mixture was allowed to cool, evaporated onto silica and purified by column chromatography to give the title compound as a white solid (0.066 g).
δ_{H} (400 MHz, CDCl₃) 2.95 (t, J = 12.0, 2H), 3.51 (m, 4H), 3.76 (t, J = 12.0, 2H), 3.82 (m, 4H), 5.05 (br s, 1H), 6.39 (s, 1H), 7.00 (s, 1H), 7.14 (h, 1H), 7.25 (m, 2H), 7.34 (m, 1H), 7.59 (m, 1H), 8.29 (br s, 1H), 8.51 (d, J = 6.4, 1H), 8.55 (s, 1H).
[M + H]⁺ 419.1.

### Examples 14 [4-(6-Fluoro-1H-indol-4-yl)-6-morpholin-4-yl-pyrimidin-2-yl]-pyridin-3-ylmethyl-amine

Prepared from 6-fluoro-4-(2-methanesulfonyl-6-morpholin-4-yl-pyrimidin-4-yl)-1H-indole (0.085 g), and 3-(aminornethyl)-pyridine (0.23 g), together with DIPEA (0.10 g), in 1,4-dioxan (2.5 mL) as described for [4-(6-fluoro-1H-indol-4-yl)-6-morpholin-4-yl-pyrimidin-2-yl]-(2-pyridin-3-yl-ethyl)-amine to give the title compound as an off-white solid (0.032 g).
δ_{H} (400 MHz, CDCl₃) 3.64 (m, 4H), 3.81 (m, 4H), 4.72 (d, J = 6.0, 2H), 5.37 (br s, 1H), 6.41 (s, 1H), 6.93 (m, 1H), 7.15 (m, 1H), 7.25 (m, 2H), 7.35 (m, 1H), 7.75 (d, J = 7.8, 1H), 8.30 (br s, 1H), 8.53 (d, J = 4.1, 1H), 8.68 (s, 1H).
[M + H]⁺ 405.2.

### Examples 15 [2-(3-Chloro-phenyl)-ethyl]-[4-(6-fluoro-1H-indol-yl)-6-morpholin-4-yl-pyrimidin-2-yl]-amine

A mixture of 6-fluoro-4-(2-methanesulfonyl-6-morpholin-4-yl-pyrimidin-4-yl)-1H-indole (75 mg, 0.20 mmol) and 3-chlorophenethylamine (310 mg, 2.00 mmol) in DIPEA (0.1 mL, 0.57 mmol) and dioxane (0.4 mL) were heated in a microwave reactor at 150°C for 40 minutes. The reaction mixture was purified by preparative HPLC to yield the title compound as an off white solid (71 mg).
δ_{H} (400MHz, CDCl₃) 2.86 (t, J = 7.1, 2H), 3.58 (t, J = 4.5, 4H), 3.66 (q, J = 6.6, 2H), 3.73 ( t, J = 4.7, 4H), 4.90 (br s, 1H), 6.29 (s, 1H), 6.91 (s, 1H), 7.06 (d, J = 8.2, 2H), 7.10-7.20 (m, 4H), 7.25 (d, J = 10.0, 1H), 8.15 (br s, 1H).
[M + H]⁺ 452.10

### Example 16 6-Fluoro-4-[6-morpholin-4-yl-2-(pyridin-3-ylmethoxy)-pyrimidin-4-yl]-1H-indole

To a stirring solution of 3-pyridylcarbinol (19.3 µL, 0.20 mmol) in DMF (1.5 mL) cooled down to 0°C under nitrogen was added sodium hydride (17.5 mg, 0.20 mmol) quickly. The suspension was stirred at 0°C for 30 minutes. 6-Fluoro-4-(2-methanesulfonyl-6-morpholin-4-yl-pyrimidin-4-yl)-1H-indole (75 mg, 0.20 mmol) was then added and the mixture warmed to room temperature. After stirring overnight, the reaction mixture was quenched with brine and extracted into ethyl acetate. The organic layers were washed with water, brine, separated and dried (MgSO₄). The crude product was purified by column chromatography to yield 6-fluoro-4-[6-morpholin-4-yl-2-(pyridin-3-ylmethoxy)-pyrimidin-4-yl]-1H-indole (30 mg).
δ_{H} (400MHz, CDCl₃) 3.73 (t, J = 4.7, 4H), 3.83 (t, J = 4.7, 4H), 5.56 (s, 2H), 6.72 (s, 1H), 6.93 (s, 1H), 7.19 (dd, J = 1.4, 7.4, 1H), 7.30 (m, 1H), 7.36-7.42 (m, 2H), 7.95 (d, J = 7.7, 1H), 8.37 (br s, 1H), 8.59 ( br d, J = 4.0, 1H), 8.79 ( s, 1H).
[M + H]⁺ 406.15

### Example 17 6-Fluoro-4-[6-morpholin-4-yl-2-(2-pyridin-3-yl-ethoxy)-pyrimidin-4-yl]-1H-indole

Prepared using method for 6-fluoro-4-[6-morpholin-4-yl-2-(pyridin-3-ylmethoxy)-pyrimidin-4-yl]-1H-indole to give an off-white solid (31 mg).
δ_{H} (400MHz, CDCl₃) 3.10 (t, J = 7.1, 2H), 3.63 (t, J = 4.4, 4H), 3.73 (t, J = 4.4, 4H), 4.57 (t, J = 7.1, 2H), 6.60 (s, 1H), 6.91 (s, 1H), 7.09 (d, J = 8.9, 1H), 7.14-7.19 (m, 2H), 7.33 (d, J = 10.6, 1H), 7.59 (d, J = 7.8, 1H), 8.28 (br s, 1H), 8.41 (d, J = 4.7, 1H), 8.51 (s, 1H).
[M + H]⁺ 420.12

### Examples 18 and 19 N-[4-(6-Fluoro-1H-indol-4-yl)-6-morpholin-4-pyrimidin-2-yl]-nicotinamide and 4-(6-Fluoro-1H-indol-4-yl)-6-morpholin-4-yl-pyrimidin-2-ylamine

To a solution of 4-chloro-6-morpholin-4-yl-pyrimidin-2-ylamine (0.180 g), stirring in anhydrous tetrahydrofuran (10 mL), was added nicotinoyl chloride HCl salt (0.299 g), followed by triethylamine (407 µL). The reaction mixture was heated at 68°C for 24 hours, then cooled and partitioned between water and dichloromethane. The combined organic extracts were dried and the solvents removed *in vacuo* to give *N*-(4-chloro-6-morpholin-4-yl-pyrimidin-2-yl)-nicotinamide as an off-white solid (0.226 g).

To a solution of 6-fluoro-4-(4, 4, 5, 5-tetramethyl-[1, 3, 2] dioxaborolan-2-yl)-1H-indole (0.110 g), with *N*-(4-chloro-6-morpholin-4-yl-pyrimidin-2-yl)-nicotinamide (0.075 g) in DME (4 mL) was added cesium fluoride (96 mg) and palladium tetrakistriphenylphosphine (18 mg). The mixture was heated in a microwave reactor at 125 °C for 20 minutes and then allowed to cool. The crude mixture was partitioned between water and dichloromethane, the combined organic layers separated and dried (MgSO₄). The crude mixture which was separated by flash silica chromatography to give the title compounds (1) as a white solid (0.028 g) and (2) as a white solid (0.005 g).
(1) δ_{H} (400 MHz, CDCl₃) 3.5 (m, 4H), 3.60 (m, 4H), 6.52 (s, 1H), 6.98 (m, 2H), 7.11 (m, 2H), 7.23 (m, 1H), 8.02 (m, 1H), 8.09 (m, 1H), 8.22 (br s, 1H), 8.57 (d, J = 4.7, 1H), 8.93 (br s, 1H).
   [M + H]⁺ 419.1
(2) δ_{H} (400 MHz, CDCl₃) 3.59 (m, 4H), 3.72 (m, 4H), 5.01 (br s, 2H), 6.32 (s, 1H), 6.84 (s, 1H), 7.06 (m, 1H), 7.24 (m, 2H), 8.24 (br s, 1H).
   [M + H]⁺ 314.1.

### Example 20 [2-(6-Methanesulfonyl-1H-indol-4-yl)-6-morpholin-4-yl-pvrimidin-4-yl]-(2-pyridin-3-yl-ethyl)-amine

4-(4-Chloro-6-morpholin-4-yl-pyrimidin-2-yl)-6-methanesulfonyl-1H-indole was prepared from 4-(6-chloro-2-iodo-pyrimidin-4-yl)-morpholine using Suzuki Method C of Reference Example 2 to give an off-white solid (43 mg).

4-(4-Chloro-6-morpholin-4-yl-pyrimidin-2-yl)-6-methanesulfonyl-1H-indole (40 mg, 0.10 mmol), 3-(2-aminoethyl)pyridine (200 mg, 0.98 mmol) and 1-methyl-2-pyrrolidinone (1 mL) were sealed in a tube and heated to 150°C overnight. The mixture was partitioned between ethyl acetate and brine, separated, and dried (MgSO₄). The crude product was purified by column chromatography to yield the title compound. (24 mg).
δ_{H} (400MHz, CDCl₃) 3.03 (t, J = 7.0, 2H), 3.16 (s, 3H), 3.67-3.72 (m, 6H), 3.86 (t, J = 4.5, 4H), 4.95 (br s, 1H), 5.41 (s, 1H), 7.28 (m, 1H), 7.56 (m, 2H), 7.65(d, J = 7.4, 1H), 8.13 (s, 1H), 8.56 (br s, 2H), 8.69 (s, 1H), 8.74 (s, 1H).
[M + H]⁺ 479.15

### Example 21 [2-(6-Fluoro-1H-indol-4-yl)-6-morpholin-4-vl-pyrimidin-4-yl]-(2-pyridin-3-yl-ethyl)-amine

Prepared using method described for [2-(6-methanesulfonyl-1H-indol-4-yl)-6-morpholin-4-yl-pyrimidin-4-yl]-(2-pyridin-3-yl-ethyl)-amine to yield an off white solid (30 mg).
δ_{H} (400MHz, CDCl₃) 3.02 (t, J = 6.9, 2H), 3.67 (t, J = 4.5, 4H), 3.72 (t, J = 6.5, 2H), 3.85(t, J = 4.5, 4H), 4.88 (br s, 1H), 5.40 (s,1H), 7.18 (d, J = 8.8, 1H), 7.28 (m, 2H), 7.46 (s, 1H), 7.61 (d, J = 7.6, 1H), 7.95 (d, J = 11.3, 1H), 8.25 (br s, 1H), 8.56 (m, 2H).
[M + H]⁺ 419.11

### Example 22 [4-(6-Fluoro-1H-indol-+yl)-6-morpholin-4-yl-pyrimidin-2-yl]-pyridin-3-yl-amine

To a stirring solution of 3-aminopyridine (60 mg, 0.63 mmol) in DMF (2 mL) cooled down to 0°C under nitrogen was added sodium hydride (37.5 mg, 0.63 mmol). The suspension was stirred at 0°C for 30 minutes. 4-(6-Chloro-2-iodo-pyrimidin-4-yl)-morpholine (200 mg, 0.63 mmol) was then added and the mixture warmed to room temperature. After stirring overnight, the reaction mixture was quenched with brine and extracted into ethyl acetate. The organic layers were washed with water, brine, separated and dried (MgSO₄). The crude product was purified by column chromatography to yield (4-chloro-6-morpholin-4-yl-pyrimidin-2-yl)-pyridin-3-yl-amine as a yellow solid (38 mg).

The title compound was prepared using Suzuki Method C of Reference Example 2 to give an off white solid (18 mg).
δ_{H} (400MHz, CDCl₃) 3.73 (t, J = 4.7, 4H), 3.86 (t, J = 4.7, 4H), 6.58 (s, 1H), 7.00 (s, 2H), 7.20 (d, J = 9.1, 1H), 7.25-7.30 (m, 2H), 7.39 (dd, J = 2.0, 8.4, 1H), 8.16 (d, J = 8.5, 1H), 8.27 (d, J = 4.5, 1H), 8.32 (br s, 1H), 8.87 (s, 1H).
[M + H]⁺ 391.11

### Example 23: [4-(6-Fluoro-1H-indol-4-yl)-6-morpholin-4-yl-pyrimidin-2-yl]-(1,2,3,4-tetrahvdro-isoquinolin-8-yl)-amine

Prepared by using Suzuki coupling method A of Reference Example 2, followed by BOC-deprotection using TFA:DCM (1:4). The title compound was obtained as a yellow foam (15 mg, 12 %).
[M + H]⁺ 445.2
¹H NMR (400 MHz, CD₃OD): δ 2.79 (t, J = 6.0 Hz, 2 H), 3.07-3.13 (m, 2 H), 3.67-3.74 (m, 4 H), 3.76-3.81 (m, 4 H), 3.95 (s, 2 H), 6.61 (s, 1 H), 6.90 (d, J = 3.3 Hz, 1 H), 7.01 (d, J = 8.3 Hz, 1 H), 7.18 (dd, J = 9.4, 2.3 Hz, 1 H), 7.28-7.33 (m, 2 H), 7.40 (dd, J = 8.3, 2.2 Hz, 1 H) and 7.55 (s, 1 H).

### Examples 24 [4-(5-Fluoro-1H-indol-4-yl)-6-morpholin-2-yl]-(1,2,3,4-tetrahydro-isoquinolin-6-yl)-amine

Prepared from 6-(4-chloro-6-morpholin-4-yl-pyrimidin-2-ylamino)-3,4-dihydro-1*H-*isoquinoline-2-carboxylic acid tert-butyl ester (156 mg; 0.35 mmol) and 5-fluoro-4-(4,4,5,5-tetramethyl-[1,3,2]dioxaborolan-2-yl)-1*H*-indole (144 mg; 0.49 mmol) using Suzuki Method A of Reference Example 2. Deprotection of the Boc-intermediate (147 mg) with TFA/CH₂Cl₂ followed by SCX-2 purification then Et₂O/MeOH trituration afforded the title compound as an off-white solid (88 mg; 73 %).
δ_{H} (400 MHz, CDCl₃) 2.79 (d, J = 6.0, 2H), 3.16 (d, J = 6.0, 2H), 3.69-3.71 (m, 4H), 3.82-3.85 (m, 4H), 4.00 (s, 2H), 6.48 (s, 1H), 6.96-7.07 (m, 4H), 7.30-7.48 (m, 3H), 8.26 (br s, 1H).
[M+H]⁺ 445.

### Example 25 [4-(6-Methanesulfonyl-1H-indol-4-yl)-6-morpholin-4-yl-pyrimidin-2-yl]-(1,2,3,4-tetrahydro-isoguinolin-6-yl)-amine

Prepared from 6-(4-chloro-6-morpholin-4-yl-pyrimidin-2-ylamino)-3,4-dihydro-1*H-*isoquinoline-2-carboxylic acid *tert*-butyl ester (156 mg; 0.35 mmol) and 6-methanesulfonyl-4-(4,4,5,5-tetramethyl-[1,3,2]dioxaborolan-2-yl)-1*H*-indole (157 mg; 0.49 mmol) using Suzuki Method B of Reference Example 2. Deprotection of the Boc-intermediate (215 mg) with TFA/CH₂Cl₂ followed by SCX-2 purification then EtOAc trituration afforded the title compound as an off-white solid (23 mg; 13 %).
δ_{H} (400 MHz, d₆-DMSO) 2.65 (t, J = 5.6, 2H), 2.94 (t, J = 5.6, 2H), 3.25 (s, 3H), 3.68-3.79 (m, 10H), 6.74 (s, 1H), 6.88 (d, J = 8.4, 1H), 7.16 (s, 1H), 7.42 (d, J = 8.4, 1H), 7.69 (s, 1H), 7.80 (s, I H), 8.07 (d, J = 8.8, 1H), 9.08 (s, 1H), 11.88 (br s, 1H).
[M+H]⁺ 505.

### Example 26 [4-(5-Fluoro-1H-indol-4-yl)-6-morpholin-4-yl-pyrimidin-2-yl]-(1,2,3,4-tetrahydro-isoquinolin-7-yl)-amine

Prepared from 7-(4-chloro-6-morpholin-4-yl-pyrimidin-2-ylamino)-3,4-dihydro-1*H-*isoquinoline-2-carboxylic acid benzyl ester (120 mg; 0.25 mmol) and 5-fluoro-4-(4,4,5,5-tetramethyl-[1,3,2]dioxaborolan-2-yl)-1*H*-indole (97 mg; 0.33 mmol) using Suzuki Method B of Reference Example 2. The Cbz-intermediate (104 mg) was dissolved in EtOH (15 ml), 10 % Pd/C (19 mg) was added and the mixture stirred under an atmosphere of hydrogen overnight (19 h). The catalyst was removed by filtration, solvent evaporated and the residue purified by SCX-2 then prep. LCMS to afford the title compound as cream-coloured solid (40 mg; 50 %).
δ_{H} (400 MHz, CDCl₃) 2.19 (s, 1H), 2.77 (t, J = 6.0, 2H), 3.16 (t, J = 6.0, 2H), 3.68-3.71 (m, 4H), 3.82-3.85 (m, 4H), 4.00 (s, 2H), 6.49 (s, 1H), 7.93-7.07 (m, 4H), 7.28-7.40 (m, 3H), 7.48 (s, 1H), 8.24 (br s, 1H).
[M+H]⁺ 445.

### Example 27 [4-(6-Fluoro-1H-indol-4-yl)-6-morpholin-4-yl-pyrimidin-2-yl]-(1,2,3,4-tetrahydro-isoquinolin-5-yl)-amine

Prepared from 5-(4-chloro-6-morpholin-4-yl-pyrimidin-2-ylamino)-3,4-dihydro-1*H-*isoquinoline-2-carboxylic acid *tert*-butyl ester (114 mg; 0.26 mmol) and 5-fluoro-4-(4,4,5,5-tetramethyl-[1,3,2]dioxaborolan-2-yl)-1*H*-indole (87 mg; 0.33 mmol) using Suzuki Method B of Reference Example 2. Deprotection of the Boc-intermediate with TFA/CH₂Cl₂ followed by SCX-2 purification then Et₂O/MeOH trituration afforded the title compound as an off-white solid (64 mg).
δ_{H} (400 MHz, CDCl₃) 1.62 (br s, 1H), 2.74 (t, J = 6.0, 2H), 3.24 (t, J = 6.0, 2H), 3.68-3.71 (m, 4H), 3.82-3.85 (m, 4H), 4.06 (s, 2H), 6.52 (s, 1H), 6.73 (s, 1H), 6.79 (d, J = 8.0, 1H), 6.99 (s, 1H), 7.15-7.22 (m, 2H), 7.26-7.28 (m, 1H), 7.39 (dd, J = 10.4 and 1.6, 1H), 8.09 (d, J = 8.0, 1H), 8.35 (br s, 1H).
[M+H]⁺ 445.

### Example 28 [4-(5-Fluoro-1H-indol-4-yl)-6-moroholin-4-yl-pyrimidin-2-yl]-(2-thiazol-2-yl-ethyl)-amine

Prepared by using Suzuki coupling method A of Reference Example 2, followed by TBAF-deprotection. The title compound was obtained as a white solid (30 mg, 30 %).
[M + H]⁺ 425.1
¹H NMR (400 MHz, CDCl₃): δ 3.35 (t, J = 6.5 Hz, 2 H), 3.61 (m, 4 H), 3.75 (m, 4 H), 3.88 (m, 2 H), 6.28 (d, J = 2.0 Hz, 1 H), 6.81 (s, 1 H), 6.94 (dd, J = 11.0, 8.8 Hz, 1 H), 7.18 (d, J = 3.3 Hz, I H), 7.22 (m, 1 H), 7.26 (m, 1 H), 7.68 (d, J = 3.4 Hz, I H) and 8.31 (bs, 1 H).

### Example 29 [4-(5-Fluoro-1H-indol-4-yl)-6-morpholin-4-yl-pyrimidin-2-yl]-[2-(6-methyl-pyridin-2-yl)-ethyl]-amine

Prepared using Suzuki coupling method A of Reference Example 2. The title compound was obtained as a white solid (21.3 mg, 22 %).
[M + H]⁺ 433.2
¹H NMR (400 MHz, CDCl₃): δ 2.54 (s, 3 H), 3.10 (t, J = 6.8 Hz, 2 H), 3.66 (m, 4 H), 3.76-3.81 (m, 4 H), 3.86 (m, 2 H), 6.26 (s, 1 H), 6.76 (s, 1 H), 6.85-6.94 (m, 1 H), 6.98 (d, J = 7.7 Hz, 1 H), 7.02 (d, J = 7.7 Hz, 1 H), 7.17-7.22 (m, 2 H), 7.48 (apparent t, J = 7.6 Hz, 1 H) and 8.84 (bs, 1 H).

### Example 30 [4-(5-Fluoro-1H-indol-4-yl)-6-morpholin-4-yl-pyrimidin-2-yl]-[2-(2-fluoro-pyridin-4-yl)-ethyl]-amine

Prepared using Suzuki coupling method A of Reference Example 2. The title compound was obtained as a white solid (9.3 mg, 23 %).
[M + H]⁺ 437.2
¹H NMR (400 MHz, CDCl₃): δ 2.96 (t, J = 7.0 Hz, 2 H), 3.63 (m, 4 H), 3.71 (m, 2 H), 3.76-3.83 (m, 4 H), 6.33 (s, 1 H), 6.80 (s, 1 H), 6.82 (s, 1 H), 6.99 (dd, J = 10.9, 8.8 Hz, 1 H), 7.04 (dd, J = 5.0, 1.8 Hz, 1 H), 7.25 (m, 1 H), 7.33 (dd, J = 8.8, 3.9 Hz, 1H), 8.05-8.11 (m, 1 H) and 8.43 (bs, 1 H).

### Example 31 [4-(5-Fluoro-1H-indol-4-yl)-6-morpholin-4-yl-pyrimidin-2-yl]-[2-(5-methoxy-pyridin-2-yl)-ethyl]-amine

Prepared using Suzuki coupling method A of Reference Example 2. The title compound was obtained as a white solid (12.3 mg, 19 %).
[M + H]⁺ 449.2
¹H NMR (400 MHz, CDCl₃): δ 3.03 (t, J = 6.7 Hz, 2 H), 3.58-3.68 (m, 4 H), 3.72-3.78 (m, 4 H), 3.75-3.84 (m, 5 H), 5.29 (bs, 1 H), 6.24 (s, 1 H), 6.80 (d, J = 3.8 Hz, 1 H), 6.94 (dd, J = 10.9, 8.8 Hz, 1 H), 7.06 (s, 1 H), 7.07 (s, 1 H), 7.20 (t, J = 2.8 Hz, 1 H), 7.26 (dd, J = 8.8, 3.8 Hz, 1 H), 8.21 (t, J = 1.8 Hz, 1 H) and 8.38 (bs, 1 H).

### Example 32 4-(6-Fluoro-1H-indol-4-yl)-6-morpholin-4-yl-pvrimidin-2-yl-(3-pyridin-3-yl-phenyl)-amine

Prepared from 4-chloro-6-morpholin-4-yl-pyrimidin-2-yl-(3-pyridin-3-yl-phenyl)-amine using Suzuki Method C of Reference Example 2. The title compound was obtained as a white solid (23 mg, 50 %)
[M+H]⁺ 467.2
δ_{H} (400 MHz, CDCl₃) 3.74 (m, 4H); 3.85 (m, 4H); 6.57 (s, 1H); 7.02 (s, 1H); 7.21 (m, 3H); 7.42 (m, 3H); 7.56 (d, 1H); 7.93 (m, 1H); 8.21 (s, 1H); 8.35 (bs, 1H); 8.61 (d, 1H); 8.89 (s, 1H).

### Example 33 Biological Testing

Compounds of the invention, prepared as described in the preceding Examples, were submitted to the following series of biological assays:

### (i) PI3K Biochemical Screening

Compound inhibition of PI3K was determined in a radiometric assay using purified, recombinant enzyme and ATP at a concentration of 1uM. All compounds were serially diluted in 100% DMSO. The kinase reaction was incubated for 1 hour at room temperature, and the reaction was terminated by the addition of PBS. IC₅₀ values were subsequently determined using sigmoidal dose-response curve fit (variable slope). All of the compounds tested had an IC₅₀ against PI3K of 50µM or less. Typically the IC₅₀ against PI3K was 5 - 500nM.

### (ii) Cellular Proliferation Inhibition

Cells were seeded at optimal density in a 96 well plate and incubated for 4 days in the presence of test compound. Alamar Blue^{™} was subsequently added to the assay medium, and cells were incubated for 6 hours before reading at 544nm excitation, 590nm emission. EC₅₀ values were calculated using a sigmoidal dose response curve fit. All the compounds tested had an EC₅₀s of 50uM or less in the range of cell lines utilized.

### Example 34 Tablet composition

Tablets, each weighing 0.15 g and containing 25 mg of a compound of the invention were manufactured as follows:
Composition for 10,000 tablets
Compound of the invention (250 g)
Lactose (800 g)
Corn starch (415g)
Talc powder (30 g)
Magnesium stearate (5 g)

The compound of the invention, lactose and half of the corn starch were mixed. The mixture was then forced through a sieve 0.5 mm mesh size. Corn starch (10 g) is suspended in warm water (90 ml). The resulting paste was used to granulate the powder. The granulate was dried and broken up into small fragments on a sieve of 1.4 mm mesh size. The remaining quantity of starch, talc and magnesium was added, carefully mixed and processed into tablets.

### Example 35 Injectable Formulation

| | |
|---|---|
| Compound of the invention | 200mg |
| Hydrochloric Acid Solution 0.1M or | |
| Sodium Hydroxide Solution 0.1M q.s. to pH | 4.0 to 7.0 |
| Sterile water q.s. to | 10 ml |

The compound of the invention was dissolved in most of the water (35°-40°C) and the pH adjusted to between 4.0 and 7.0 with the hydrochloric acid or the sodium hydroxide as appropriate. The batch was then made up to volume with water and filtered through a sterile micropore filter into a sterile 10 ml amber glass vial (type 1) and sealed with sterile closures and overseals.

### Example 36 Intramuscular Injection

| | |
|---|---|
| Compound of the invention | 200 mg |
| Benzyl Alcohol | 0.10 g |
| Glycofurol 75 | 1.45 g |
| Water for injection q.s to | 3.00 ml |

The compound of the invention was dissolved in the glycofurol. The benzyl alcohol was then added and dissolved, and water added to 3 ml. The mixture was then filtered through a sterile micropore filter and sealed in sterile 3 ml glass vials (type 1).

### Example 37 Syrup Formulation

| | |
|---|---|
| Compound of invention | 250 mg |
| Sorbitol Solution | 1.50 g |
| Glycerol | 2.00 g |
| Sodium benzoate | 0.005 g |
| Flavour | 0.0125 ml |
| Purified Water q.s. to | 5.00 ml |

The compound of the invention was dissolved in a mixture of the glycerol and most of the purified water. An aqueous solution of the sodium benzoate was then added to the solution, followed by addition of the sorbital solution and finally the flavour. The volume was made up with purified water and mixed well.

## Claims

1. A compound which is a pyrimidine of formula (I): 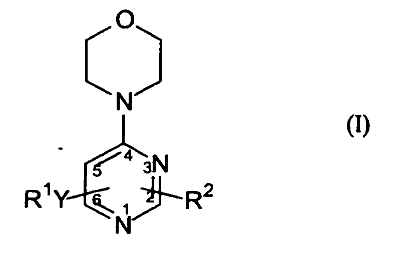 wherein
R² is bonded at ring position 2 and -YR¹ is bonded at ring position 5 or 6, or YR¹ is bonded at ring position 2 and R² is bonded at ring position 6;
R² is an indol-4-yl group which is substituted at the 5- or 6-position;
either:
(a) Y is selected from -O-(CH₂)ₙ-, -NH-(CH₂)ₙ-, -NHC(O)-(CH₂)ₙ- and -C(O)NH-(CH₂)- wherein n is 0 or an integer of 1 to 3, and R¹ is selected from an unsaturated 5- to 12-membered carbocyclic or heterocyclic group which is unsubstituted or substituted and a group -NR³R⁴ wherein R³ and R⁴, which are the same or different, are each independently selected from H, C₁-C₆ alkyl which is unsubstituted or substituted, C₃ - C₁₀ cycloalkyl which is unsubstituted or substituted, -C(O)R, -C(O)N(R)₂ and -S(O)ₘR, or R³ and R⁴ together form, with the nitrogen atom to which they are attached, a saturated 5-, 6- or 7-membered N-containing heterocyclic group which is unsubstituted or substituted;
(b) Y is a direct bond and R¹ is selected from an unsaturated 5- to 12-membered carbocyclic or heterocyclic group which is unsubstituted or substituted, and a group -NR³R⁴ wherein R³ and R⁴, which are the same or different, are each independently selected from H, C₁-C₆ alkyl which is unsubstituted or substituted, C₃-C₁₀ cycloalkyl which is unsubstituted or substituted, -C(O)R, -C(O)N(R)₂ and -S(O)ₘR;
R is selected from H, C₁-C₆ alkyl, C₃-C₁₀ cycloalkyl and a 5- to 12-membered aryl or heteroaryl group, which group is unsubstituted or substituted; and
m is 1 or 2;
or a pharmaceutically acceptable salt thereof.

2. A compound according to claim 1 wherein the pyrimidine is of formula (Ia): 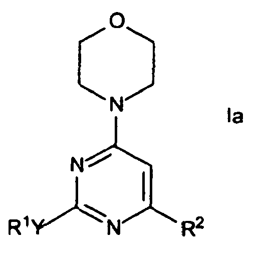 in which R¹, R², and Y are as defined in claim 1.

3. A compound according to claim 1 wherein the pyrimidine is of formula (Ib): 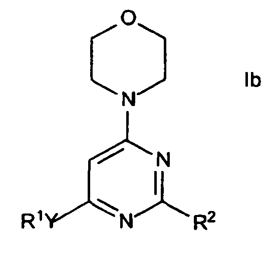 in which R¹, R², and Y are as defined in claim 1.

4. A compound according to claim 1 wherein the pyrimidine is of formula (Ic): 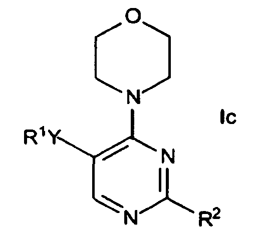 in which R¹, R², and Y are as defined in claim 1.

5. A compound which is selected from:
4-(6-Fluoro-1*H*-indol-4-yl)-6-morpholin-4-yl-pyrimidine-2-carboxylic acid (2-dimethylamino-ethyl)-amide;
4-(6-Fluoro-1*H*-indol-4-yl)-6-morpholin-4-yl-pyrimidine-2-carboxylic acid pyridin-3-ylamide;
4-[6-Morpholin-4-yl-2-(2-pyridin-3-yl-ethylamino)-pyrimidin-4-yl]-1H-indole-6-carbonitrile;
[4-(6-Methanesulfonyl-1H-indol-4-yl)-6-morpholin-4-yl-pyrimidin-2-yl]-(2-pyridin-3-yl-ethyl)-amine;
4-[6-Morpholin-4-yl-2-(2-pyridin-3-yl-ethylamino)-pyrimidin-4-yl]-1H-indole-6-sulfonic acid dimethylamide;
4-[6-Morpholin-4-yl-2-(2-pyridin-3-yl-ethylamino)-pyrimidin-4-yl]-1H-indole-6-carboxylic acid amide;
[4-Morpholin-4-yl-6-(6-trifluoromethyl-1H-indol-4-yl)-pyrimidin-2-yl]-(2-pyridin-3-yl-ethyl)-amine;
[4-(5-Fluoro-1H-indol-4-yl)-6-morpholin-4-yl-pyrimidin-2-yl]-(2-pyridin-3-yl-ethyl)-amine;
6-Fluoro-4-(6-morpholin-4-yl-2-phenyl-pyrimidin-4-yl)-1H-indole;
6-Fluoro-4-(6-morpholin-4-yl-2-pyridin-3-yl-pyrimidin-4-yl)-1H-indole;
6-Fluoro-4-(6-morpholin-4-yl-2-pyridin-4-yl-pyrimidin-4-yl)-1H-indole;
6-Fluoro-4-[6-morpholin-4-yl-2-(pyridin-3-yloxy)-pyrimidin-4-yl]-1H-indole;
[4-(6-Fluoro-1H-indol-4-yl)-6-morpholin-4-yl-pyrimidin-2-yl]-(2-pyridin-3-yl-ethyl)-amine;
[4-(6-Fluoro-1H-indol-4-yl)-6-morpholin-4-yl-pyrimidin-2-yl]-pyridin-3-ylmethyl-amine;
[2-(3-Chloro-phenyl)-ethyl]-[4-(6-fluoro-1H-indol-4-yl)-6-morpholin-4-yl-pyrimidin-2-yl]-amine;
6-Fluoro-4-[6-morpholin-4-yl-2-(pyridin-3-ylmethoxy)-pyrimidin-4-yl]-1H-indole;
6-Fluoro-4-[6-morpholin-4-yl-2-(2-pyridin-3-yl-ethoxy)-pyrimidin-4-yl]-1H-indole;
*N*-[4-(6-Fluoro-1H-indol-4-yl)-6-morpholin-4-yl-pyrimidin-2-yl]-nicotinamide;
4-(6-Fluoro-1H-indol-4-yl)-6-morpholin-4-yl-pyrimidin-2-ylamine;
[2-(6-Methanesulfonyl-1H-indol-4-yl)-6-morpholin-4-yl-pyrimidin-4-yl]-(2-pyridin-3-yl-ethyl)-amine;
[2-(6-Fluoro-1H-indol-4-yl)-6-morpholin-4-yl-pyrimidin-4-yl]-(2-pyridin-3-yl-ethyl)-amine;
[4-(6-Fluoro-1H-indol-4-yl)-6-morpholin-4-yl-pyrimidin-2-yl]-pyridin-3-yl-amine;
[4-(6-Fluoro-1*H*-indol-4-yl)-6-morpholin-4-yl-pyrimidin-2-yl]-(1,2,3,4-tetrahydro-isoquinolin-7-yl)-amine;
[4-(5-Fluoro-1*H*-indol-4-yl)-6-morpholin-4-yl-pyrimidin-2-yl]-(1,2,3,4-tetrahydro-isoquinolin-6-yl)-amine;
[4-(6-Methanesulfonyl-1*H*-indol-4-yl)-6-morpholin-4-yl-pyrimidin-2-yl]-(1,2,3,4-tetrahydro-isoquinolin-6-yl)-amine;
[4-(5-Fluoro-1*H*-indol-4-yl)-6-morpholin-4-yl-pyrimidin-2-yl]-(1,2,3,4-tetrahydro-isoquinolin-7-yl)-amine;
[4-(6-Fluoro-1*H-*indol-4-yl)-6-morpholin-4-yl-pyrimidin-2-yl]-(1,2,3,4-tetrahydro-isoquinolin-5-yl)-amine;
[4-(5-Fluorc-1*H*-indol-4-yl)-6-morpholin-4-yi-pyrimidin-2-yl]-(2-thiazol-2-yl-ethyl)-ainine;
[4-(5-Fluoro-1*H*-indol-4-yl)-6-morpholin-4-yl-pyrimidin-2-yl]-[2-(6-methyl-pyridin-2-yl)-ethyl]-amine;
[4-(5-Fluoro-1*H*-indol-4-yl)-6-morphotin-4-yl-pyrimidin-2-yl]-[2-(2-fluoro-pyridin-4-yl)-ethyl]-amine;
[4-(5-Fluoro-1*H*-indol-4-yl)-6-morpholin-4-yl-pyrimidin-2-yl]-[2-(5-methoxy-pyridin-2-yl)-ethyl]-amine;
4-(6-Fluoro-1H-indol-4-yl)-6-morpholin-4-yl-pyrimidin-2-yl-(3-pyridin-3-yl-phenyl)-amine; and the pharmaceutically acceptable salts thereof.

6. A pharmaceutical composition which comprises a pharmaceutically acceptable carrier or diluent and, as an active ingredient, a compound as defined in any one of claims 1 to 5.

7. A compound as defined in any one of claims 1 to 5 for use in a method of medical treatment of the human or animal body by therapy.

8. A compound as defined in any one of claims 1 to 5, for use in treating cancer, immune disorders, cardiovascular disease, viral infection, inflammation, metabolism/endocrine function disorders or neurological disorders

9. Use of a compound as defined in any one of claims 1 to 5 in the manufacture of a medicament for treating cancer, immune disorders, cardiovascular disease, viral infection, inflammation, metabolism/endocrine function disorders or neurological disorders.

## Patentansprüche

1. Eine Verbindung, die ein Pyrimidin ist der Formel (I): 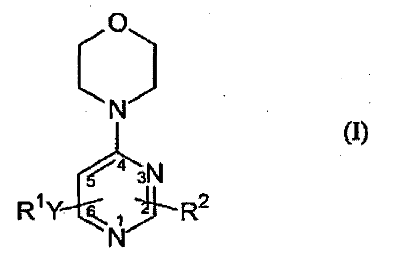 wobei
R² an Ringposition 2 gebunden ist und -YR¹ an Ringposition 5 oder 6 gebunden ist,
oder YR¹ an Ringposition 2 gebunden ist und R² an Ringposition 6 gebunden ist;
R² eine Indol-4-yl Gruppe ist, die an der 5- oder 6-Position substituiert ist;
entweder:
(a) Y ausgewählt ist aus -O-(CH₂)ₙ-, -NH-(CH₂)ₙ-, -NHC(O)-(CH₂)ₙ- und -C(O)NH-(CH₂)ₙ- wobei n gleich 0 oder eine ganze Zahl von 1 bis 3 ist, und R¹ ausgewählt ist aus einer ungesättigten 5- bis 12-gliedrigen carbocyclischen oder heterocyclischen Gruppe, die unsubstituiert oder substituiert ist, und einer Gruppe -NR³R⁴, wobei R³ und R⁴, die gleich oder verschieden sind, jeder unabhängig ausgewählt sind aus H, C₁-C₆ Alkyl, das unsubstituiert oder substituiert ist, C₃ - C₁₀ Cycloalkyl, das unsubstituiert oder substituiert ist, -C(O)R, -C(O)N(R)₂ und -S(O)ₘR, oder R³ und R⁴ zusammen mit dem Stickstoffatom, an das sie gebunden sind, eine gesättigte 5-, 6- oder 7- gliedrige N-haltige heterocyclische Gruppe bilden, die unsubstituiert oder substituiert ist;
(b) Y eine direkte Bindung ist und R¹ ausgewählt ist aus einer ungesättigten 5- bis 12-gliedrigen carbocyclischen oder heterocyclischen Gruppe, die unsubstituiert oder substituiert ist, und einer Gruppe -NR³R⁴, wobei R³ und R⁴, die gleich oder verschieden sind, jeder unabhängig ausgewählt sind aus H, C₁-C₆ Alkyl, das unsubstituiert oder substituiert ist, C₃ - C₁₀ Cycloalkyl, das unsubstituiert oder substituiert ist, -C(O)R, -C(O)N(R)₂ und -S(O)ₘR;
R ausgewählt ist aus H, C₁-C₆ Alkyl, C₃-C₁₀ Cycloalkyl und einer 5- bis 12-gliedrigen Aryl oder Heteroarylgruppe, die unsubstituiert oder substituiert ist; und
m gleich 1 oder 2 ist;
oder ein pharmazeutisch verträgliches Salz davon.

2. Eine Verbindung nach Anspruch 1, wobei das Pyrimidin die Formel (la) hat: 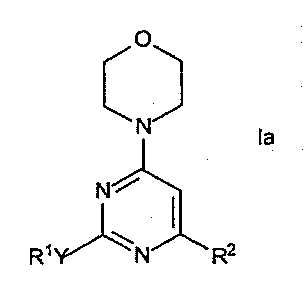 in der R¹, R² und Y wie in Anspruch 1 definiert sind.

3. Eine Verbindung nach Anspruch 1, wobei das Pyrimidin die Formel (Ib) hat: 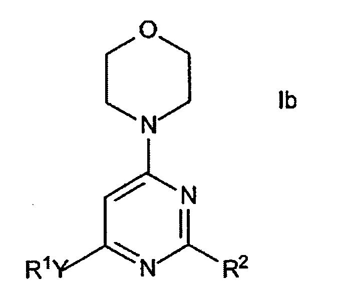 in der R¹, R² und Y wie in Anspruch 1 definiert sind.

4. Eine Verbindung nach Anspruch 1, wobei das Pyrimidin die Formel (Ic) hat: 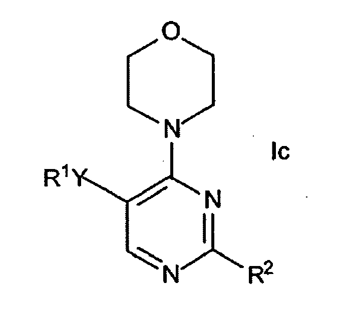 in der R¹, R² und Y wie in Anspruch 1 definiert sind.

5. Eine Verbindung, die ausgewählt ist aus:
4-(6-Fluor-1*H*-indol-4-yl)-6-morpholin-4-yl-pyrimidin-2-carbonsäure (2-dimethylamino-ethyl)-amid;
4-(6-Fluor-1*H*-indol-4-yl)-6-morpholin-4-yl-pyrimidin-2-carbonsäure pyridin-3-ylamid;
4-[6-Morpholin-4-yl-2-(2-pyridin-3-yl-ethylamino)-pyrimidin-4-yl]-1H-indol-6-carbonitril;
[4-(6-Methansulfonyl-1H-indol-4-yl)-6-morpholin-4-yl-pyrimidin-2-yl]-(2-pyridin-3-yl-ethyl)-amin;
4-[6-Morpholin-4-yl-2-(2-pyridin-3-yl-ethylamino)-pyrimidin-4-yl]-1H-indol-6-sulfonsäure dimethylamid;
4-[6-Morpholin-4-yl-2-(2-pyridin-3-yl-ethylamino)-pyrimidin-4-yl]-1H-indol-6-carbonsäure amid;
[4-Morpholin-4-yl-6-(6-trifluormethyl-1H-indol-4-yl)-pyrimidin-2-yl]-(2-pyridin-3-yl-ethyl)-amin;
[4-(5-Fluor-1H-indol-4-yl)-6-morpholin-4-yl-pyrimidin-2-yl]-(2-pyridin-3-yl-ethyl)-amin;
6-Fluor-4-(6-morpholin-4-yl-2-phenyl-pyrimidin-4-yl)-1H-indol;
6-Fluor-4-(6-morpholin-4-yl-2-pyridin-3-yl-pyrimidin-4-yl)-1H-indol;
6-Fluor-4-(6-morpholin-4-yl-2-pyridin-4-yl-pyrimidin-4-yl)-1H-indol;
6-Fluor-4-[6-morpholin-4-yl-2-(pyridin-3-yloxy)-pyrimidin-4-yl]-1H-indol;
[4-(6-Fluor-1H-indol-4-yl)-6-morpholin-4-yl-pyrimidin-2-yl]-(2-pyridin-3-yl-ethyl)-amin;
[4-(6-Fluor-1H-indol-4-yl)-6-morpholin-4-yl-pyrimidin-2-yl]-pyridin-3-ylmethyl-amin;
[2-(3-Chlor-phenyl)-ethyl]-[4-(6-fluor-1H-indol-4-yl)-6-morpholin-4-yl-pyrimidin-2-yl]-amin;
6-Fluor-4-[6-morpholin-4-yl-2-(pyridin-3-ylmethoxy)-pyrimidin-4-yl]-1H-indol;
6-Fluor-4-[6-morpholin-4-yl-2-(2-pyridin-3-yl-ethoxy)-pyrimidin-4-yl]-1H-indol;
*N*-[4-(6-Fluor-1H-indol-4-yl)-6-morpholin-4-yl-pyrimidin-2-yl]-nicotinamid;
4-(6-Fluor-1H-indol-4-yl)-6-morpholin-4-yl-pyrimidin-2-ylamin;
[2-(6-Methansulfonyl-1H-indol-4-yl)-6-morpholin-4-yl-pyrimidin-4-yl]-(2-pyridin-3-yl-ethyl)-amin;
[2-(6-Fluor-1H-indol-4-yl)-6-morpholin-4-yl-pyrimidin-4-yl]-(2-pyridin-3-yl-ethyl)-amin;
[4-(6-Fluor-1H-indol-4-yl)-6-morpholin-4-yl-pyrimidin-2-yl]-pyridin-3-yl-amin;
[4-(6-Fluor-1*H*-indol-4-yl)-6-morpholin-4-yl-pyrimidin-2-yl]-(1,2,3,4-tetrahydro-isochinolin-7-yl)-amin;
[4-(5-Fluor-1*H*-indol-4-yl)-6-morpholin-4-yl-pyrimidin-2-yl]-(1,2,3,4-tetrahydro-isochinolin-6-yl)-amin;
[4-(6-Methansulfonyl-1*H*-indol-4-yl)-6-morpholin-4-yl-pyrimidin-2-yl]-(1,2,3,4-tetrahydroisochinolin-6-yl)-amin;
[4-(5-Fluor-1*H*-indol-4-yl)-6-morpholin-4-yl-pyrimidin-2-yl]-(1,2,3,4-tetrahydro-isochinolin-7-yl)-amin;
[4-(6-Fluor-1*H*-indol-4-yl)-6-morpholin-4-yl-pyrimidin-2-yl]-(1,2,3,4-tetrahydro-isochinolin-5-yl)-amin;
[4-(5-Fluor-1*H*-indol-4-yl)-6-morpholin-4-yl-pyrimidin-2-yl]-(2-thiazol-2-yl-ethyl)-amin;
[4-(5-Fluor-1*H*-indol-4-yl)-6-morpholin-4-yl-pyrimidin-2-yl]-[2-(6-methyl-pyridin-2-yl)-ethyl]-amin;
[4-(5-Fluor-1*H*-indol-4-yl)-6-morpholin-4-yl-pyrimidin-2-yl]-[2-(2-fluor-pyridin-4-yl)-ethyl]-amin;
[4-(5-Fluor-1*H*-indol-4-yl)-6-morpholin-4-yl-pyrimidin-2-yl]-[2-(5-methoxy-pyridin-2-yl)-ethyl]-amin;
4-(6-Fluor-1*H*-indol-4-yl)-6-morpholin-4-yl-pyrimidin-2-yl-(3-pyridin-3-yl-phenyl)-amin;
und die pharmazeutisch verträglichen Salze davon.

6. Eine pharmazeutische Zusammensetzung, die einen pharmazeutisch verträglichen Trägerstoff oder Verdünnungsmittel und, als aktiven Inhaltsstoff, eine Verbindung umfasst, wie in einem der Ansprüche 1 bis 5 definiert.

7. Eine Verbindung, wie in einem der Ansprüche 1 bis 5 definiert, zur Verwendung in einem Verfahren der medizinischen Behandlung des menschlichen oder tierischen Körpers durch Therapie.

8. Eine Verbindung, wie in einem der Ansprüche 1 bis 5 definiert, zur Verwendung bei der Behandlung von Krebs, Immunstörungen, Herz-Kreislauf-Erkrankung, Virusinfektion, Entzündung, Metabolismus/endokrinen Funktionsstörungen oder neurologischen Störungen.

9. Verwendung einer Verbindung, wie in einem der Ansprüche 1 bis 5 definiert, zur Herstellung eines Arzneimittels zur Behandlung von Krebs, Immunstörungen, Herz-Kreislauf-Erkrankung, Virusinfektion, Entzündung, Metabolismus/endokrinen Funktionsstörungen oder neurologischen Störungen.

## Revendications

1. Composé qui est une pyrimidine de formule (I) : 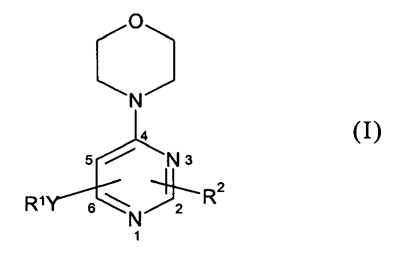 dans laquelle
le substituant R² est lié au cycle en position 2 et le substituant YR¹ est lié au cycle en position 5 ou 6, ou bien le substituant YR¹ est lié au cycle en position 2 et le substituant R² est lié au cycle en position 6 ;
R² représente un groupe indol-4-yle qui porte un substituant en position 5 ou 6 ; et
a) soit Y représente un fragment choisi parmi ceux qui sont symbolisés par -O-(CH₂)ₙ-, -NH-(CH₂)ₙ-, -NH-C(O)-(CH₂)ₙ- et -C(O)-NH-(CH₂)ₙ-, où l'indice n est nul ou est un entier valant de 1 à 3, et R¹ représente une entité choisie parmi un groupe carbocyclique ou hétérocyclique insaturé comportant de 5 à 12 chaînons, avec ou sans substituant(s), et un groupe symbolisé par -NR³R⁴, où R³ et R⁴ représentent des entités identiques ou différentes et choisies, chacune indépendamment, parmi un atome d'hydrogène, un groupe alkyle en C₁₋₆ avec ou sans substituant(s), un groupe cycloalkyle en C₃₋₁₀ avec ou sans substituant(s), et un groupe symbolisé par -C(O)R, -C(O)NR₂ ou -S(O)ₘR, ou bien R³ et R⁴ représentent des entités qui constituent, conjointement avec l'atome d'azote auquel elles sont liées, un groupe hétérocyclique azoté saturé comportant 5, 6 ou 7 chaînons, avec ou sans substituant(s) ;
b) soit Y représente une liaison directe et R¹ représente une entité choisie parmi un groupe carbocyclique ou hétérocyclique insaturé comportant de 5 à 12 chaînons, avec ou sans substituant(s), et un groupe symbolisé par -NR³R⁴ où R³ et R⁴ représentent des entités identiques ou différentes et choisies, chacune indépendamment, parmi un atome d'hydrogène, un groupe alkyle en C₁₋₆ avec ou sans substituant(s), un groupe cycloalkyle en C₃₋₁₀ avec ou sans substituant(s), et un groupe symbolisé par -C(O)R, -C(O)NR₂ ou -S(O)ₘR,
étant entendu que R représente une entité choisie parmi un atome d'hydrogène, un groupe alkyle en C₁₋₆, un groupe cycloalkyle en C₃₋₁₀, et un groupe aryle ou hétéroaryle comportant de 5 à 12 chaînons, avec ou sans substituant(s), et que l'indice m vaut 1 ou 2 ;
ou sel pharmacologiquement admissible d'un tel composé.

2. Composé conforme à la revendication 1, qui est une pyrimidine de formule (Ia) : 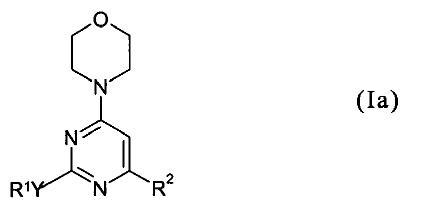 dans laquelle R¹, R² et Y ont les significations indiquées dans la revendication 1.

3. Composé conforme à la revendication 1, qui est une pyrimidine de formule (Ib) : 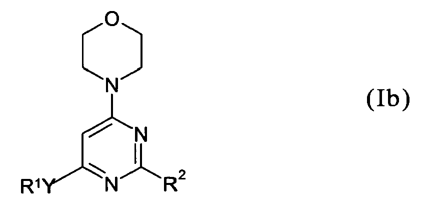 dans laquelle R¹, R² et Y ont les significations indiquées dans la revendication 1.

4. Composé conforme à la revendication 1, qui est une pyrimidine de formule (Ic) : 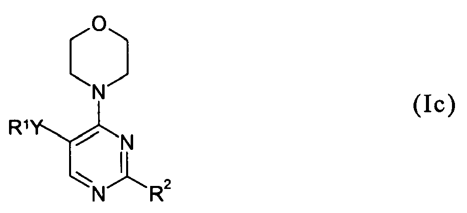 dans laquelle R¹, R² et Y ont les significations indiquées dans la revendication 1.

5. Composé qui est choisi parmi les suivants :
(2-diméthylamino-éthyl)-amide d'acide 4-(6-fluoro-1H-indol-4-yl)-6-(morpholin-4-yl)-pyrimidine-2-carboxylique
(pyridin-3-yl)-amide d'acide 4-(6-fluoro-1H-indol-4-yl)-6-(morpholin-4-yl)-pyrimidine-2-carboxylique
4-{6-(morpholin-4-yl)-2-[2-(pyridin-3-yl)-éthyl-amino]-pyrimidin-4-yl}-1H-indole-6-carbonitrile
[4-(6-méthanesulfonyl-1H-indol-4-yl)-6-(morpholin-4-yl)-pyrimidin-2-yl]-[2-(pyridin-3-yl)-éthyl]-amine
diméthyl-amide d'acide 4-{6-(morpholin-4-yl)-2-[2-(pyridin-3-yl)-éthyl-amino]-pyrimidin-4-yl}-1H-indole-6-sulfonique
amide d'acide 4-{6-(morpholin-4-yl)-2-[2-(pyridin-3-yl)-éthyl-amino]-pyrimidin-4-yl}-1H-indole-6-carboxylique
[4-(morpholin-4-yl)-6-(6-trifluorométhyl-1H-indol-4-yl)-pyrimidin-2-yl]-[2-(pyridin-3-yl)-éthyl]-amine
[4-(5-fluoro-1H-indol-4-yl)-6-(morpholin-4-yl)-pyrimidin-2-yl]-[2-(pyridin-3-yl)-éthyl]-amine
6-fluoro-4-[6-(morpholin-4-yl)-2-phényl-pyrimidin-4-yl]-1H-indole
6-fluoro-4-[6-(morpholin-4-yl)-2-(pyridin-3-yl)-pyrimidin-4-yl]-1H-indole
6-fluoro-4-[6-(morpholin-4-yl)-2-(pyridin-4-yl)-pyrimidin-4-yl]-1H-indole
6-fluoro-4-[6-(morpholin-4-yl)-2-(pyridin-3-yl-oxy)-pyrimidin-4-yl]-1H-indole
[4-(6-fluoro-1H-indol-4-yl)-6-(morpholin-4-yl)-pyrimidin-2-yl]-[2-(pyridin-3-yl)-éthyl]-amine
[4-(6-fluoro-1H-indol-4-yl)-6-(morpholin-4-yl)-pyrimidin-2-yl]-[(pyridin-3-yl)-méthyl]-amine
[2-(3-chloro-phényl)-éthyl]-[4-(6-fluoro-1H-indol-4-yl)-6-(morpholin-4-yl)-pyrimidin-2-yl]-amine
6-fluoro-4-[6-(morpholin-4-yl)-2-(pyridin-3-yl-méthoxy)-pyrimidin-4-yl]-IH-indole
6-fluoro-4-{6-(morpholin-4-yl)-2-[2-(pyridin-3-yl)-éthoxy]-pyrimidin-4-yl}-1H-indole
N-[4-(6-fluoro-1H-indol-4-yl)-6-(morpholin-4-yl)-pyrimidin-2-yl]-nicotinamide
[4-(6-fluoro-1H-indol-4-yl)-6-(morpholin-4-yl)-pyrimidin-2-yl]-amine [2-(6-méthanesulfonyl-1H-indol-4-yl)-6-(morpholin-4-yl)-pyrimidin-4-yl]-[2-(pyridin-3-yl)-éthyl]-amine
[2-(6-fluoro-1H-indol-4-yl)-6-(morpholin-4-yl)-pyrimidin-4-yl]-[2-(pyridin-3-yl)-éthyl]-amine
[4-(6-fluoro-1H-indol-4-yl)-6-(morpholin-4-yl)-pyrimidin-2-yl]-(pyridin-3-yl)-amine
[4-(6-fluoro-1H-indol-4-yl)-6-(morpholin-4-yl)-pyrimidin-2-yl]-(1,2,3,4-tétrahydro-isoquinoléin-7-yl)-amine
[4-(5-fluoro-1H-indol-4-yl)-6-(morpholin-4-yl)-pyrimidin-2-yl]-(1,2,3,4-tétrahydro-isoquinoléin-6-yl)-amine
[4-(6-méthanesulfonyl-1H-indol-4-yl)-6-(morpholin-4-yl)-pyrimidin-2-yl]-(1,2,3,4-tétrahydro-isoquinoléin-6-yl)-amine
[4-(5-fluoro-1H-indol-4-yl)-6-(morpholin-4-yl)-pyrimidin-2-yl]-(1,2,3,4-tétrahydro-isoquinoléin-7-yl)-amine
[4-(6-fluoro-1H-indol-4-yl)-6-(morpholin-4-yl)-pyrimidin-2-yl]-(1,2,3,4-tétrahydro-isoquinoléin-5-yl)-amine
[4-(5-fluoro-1H-indol-4-yl)-6-(morpholin-4-yl)-pyrimidin-2-yl]-[2-(thiazol-2-yl)-éthyl]-amine
[4-(5-fluoro-1H-indol-4-yl)-6-(morpholin-4-yl)-pyrimidin-2-yl]-[2-(6-méthyl-pyridin-2-yl)-éthyl]-amine
[4-(5-fluoro-1H-indol-4-yl)-6-(morpholin-4-yl)-pyrimidin-2-yl]-[2-(2-fluoro-pyridin-4-yl)-éthyl]-amine
[4-(5-fluoro-1H-indol-4-yl)-6-(morpholin-4-yl)-pyrimidin-2-yl]-[2-(5-méthoxy-pyridin-2-yl)-éthyl]-amine
[4-(6-fluoro-1H-indol-4-yl)-6-(morpholin-4-yl)-pyrimidin-2-yl]-[3-(pyridin-3-yl)-phényl]-amine
et leurs sels pharmacologiquement admissibles.

6. Composition pharmaceutique, comprenant un véhicule ou diluant pharmacologiquement admissible, et comme ingrédient actif, un composé conforme à l'une des revendications 1 à 5.

7. Composé conforme à l'une des revendications 1 à 5, pour son utilisation dans un procédé de traitement médical du corps d'un humain ou d'un animal par thérapie.

8. Composé conforme à l'une des revendications 1 à 5, pour son utilisation dans le traitement d'un cancer, de troubles de l'immunité, d'une maladie cardiovasculaire, d'une infection virale, d'une inflammation, de troubles du métabolisme ou d'une fonction endocrinienne, ou de troubles neurologiques.

9. Utilisation d'un composé, conforme à l'une des revendications 1 à 5, dans la fabrication d'un médicament conçu pour traiter un cancer, des troubles de l'immunité, une maladie cardiovasculaire, une infection virale, une inflammation, des troubles du métabolisme ou d'une fonction endocrinienne, ou des troubles neurologiques.
